# EUROPEAN PATENT APPLICATION

(11) **EP 4 759 311 A1**
(43) Date of publication of application: **17.06.2026**
(21) Application number: 24219914.9
(22) Date of filing: 13.12.2024
(51) Int. Cl.: A61K 31/426, A61K 9/00, A61P 19/02

(54) **TRPM8 INHIBITORS FOR USE IN THE PREVENTION OR TREATMENT OF JOINT DAMAGE, JOINT DEGENERATION AND/OR JOINT INFLAMMATION CAUSED BY OSTEOARTHRITIS**

(71) Applicant: Dompé farmaceutici SpA, 20122 Milano (IT)
(72) Inventor: CYPRIANO DUTRA, Rafael, 80131 Napli (IT); TOMASSETTI, Mara, 80131 Napoli (IT); BIANCHINI, Gianluca, 67100 L'Aquila (IT); ROCCHIO, Francesca, 80131 Napoli (IT); DETTA, Nicola, 80131 Napoli (IT); CONTE, Gemma, 80131 Napoli (IT); GIORGIO, Cristina, 67100 L'Aquila (IT); ARAMINI, Andrea, 67100 L'Aquila (IT)
(74) Representative: Dompé farmaceutici Spa

(57) **Abstract**

The invention relates to TRPM8 inhibitors for use in the prevention or treatment of joint damage and/or joint degeneration and/or joint inflammation in a subject with osteoarthritis.

## Description

### TECHNICAL FIELD

The invention relates to TRPM8 inhibitors for use in the prevention or treatment of joint damage and/or joint degeneration and/or joint inflammation caused by osteoarthritis. The invention is also directed to TRPM8 inhibitors for use in the prevention or treatment of joint damage and/or joint degeneration and/or joint inflammation in a subject with osteoarthritis.

### BACKGROUND OF THE INVENTION

Osteoarthritis (OA) is a chronic, commonly age-related degenerative disease of the joint tissue, including articular cartilage, subchondral bone, and synovium. The damage of the joint tissue results in pain and in decreased function of the affected joint (Mintarjo JA, et al., Cureus. 2023 Jun 26;15(6):e40966). Beyond aging, other OA risk factors include being overweight and different types of injuries or diseases, such as infection, autoimmune processes, degenerative processes, and trauma. Its occurrence is estimated at 22.9% in persons over 40 years of age in 2020 (Cui A, et al., EClinicalMedicine 2020; 29-30: 100587).

The knee is a frequent site for OA (Uivaraseanu B, et al. Exp Ther Med. 2022;23:328; Hamood R, et al., J Clin Med. 2021;1 0:4282; Oo WM et al., Ther Adv Musculoskelet Dis. 2022 May 20; 14: 1759720X221 090297). Knee osteoarthritis (KOA) can cause joint pain, muscle weakness, physical disability, and decreased quality of life. Nearly half of the cases of OA are KOA, and its prevalence increases with age and degree of obesity.

Despite the enormous impact of OA in patients, the treatment of OA is currently focused on symptomatic improvement only, especially in the management of pain (Bannuru RR et al., Osteoarthritis Cartilage 2019; 27: 1578-1589) and is thus mostly palliative. The pharmacologic treatment is primarily performed with analgesics, NSAIDs and opioids (Hochberg MC. Osteoarthritis Cartilage 2012; 20(12):1465-1469; Hochberg MC et al., Arthritis Care Res. (Hoboken) 2012; 64(4):465-474; Jordan KM, et al., Ann Rheum Dis 2003; 62(12):1145-1155). When the pharmacologic treatment is not effective at controlling the symptoms, surgery is required to replace the damaged joint (Bajpayee AG, et al., Osteoarthritis Cartilage 2016; 24(1):71-81; Han Y, et al., Pain Med 2019; 20(7):1418-1429; Jansen MP, et al., Cartilage 2021; 12(2):181-191). Unfortunately, surgery is often a temporary solution, and revision surgeries need to be performed as the time passes (Rodriguez-Merchan, Arch Bone Jt Surg. 2023; 11(1): 11-22).

Currently, no effective disease-modifying osteoarthritis drugs (DMOADs), i.e., drugs able to delay or reverse the progression of the structural damage of the joint, have been approved by regulatory bodies (Oo WM et al., Drug Des Devel Ther 2021; 15: 2921-2945; Rodriguez-Merchan, Arch Bone Jt Surg. 2023; 11(1): 11-22). The clinical trials published so far have produced disappointing outcomes, given that the medications have only been effective in the short term or have shown no efficacy (Chevalier X, et al., Nat Rev Rheumatol. 2013; 9(7):400-410; Rodriguez-Merchan, Arch Bone Jt Surg. 2023; 11(1): 11-22).

It is therefore felt the need to develop new therapies able to prevent, delay, block or reverse the progression of structural damage and degeneration of the joint, and associated tissues, caused by osteoarthritis.

Transient Receptor Potential (TRP) channels are one of the largest groups of ion channels and, based on their sequence homology, are classified into 6 sub-families (TRPV, TRPM, TRPA, TRPC, TRPP and TRPML). TRP channels are cation-selective channels activated by several physical (such as temperature, osmolarity and mechanical stimuli) and chemical stimuli. TRPM8, which was cloned in 2002, is a non-selective cation channel of the TRP family expressed on a subpopulation of somatic sensory nerves on dorsal root ganglion and trigeminal ganglia that causes sensory nerve excitation. It is activated by mild cold temperatures and synthetic cool-mimetic compounds such as menthol, eucalyptol and icilin (McKemy D.D. et al., Nature (2002) 416, 52-58; Peier A.M. et al. Cell (2002) 108, 705-715). Like several other TRP channels, TRPM8 is also gated by voltage (Nilius B. et al., J. Physiol. (2005) 567, 35-44). The voltage dependence of TRPM8 is characterized by a strong outward rectification at depolarized transmembrane potential and a rapid and potential-dependent closure at negative membrane potentials. Cooling agents and menthol application shifts the activation curve towards more negative potentials, increasing the possibility for the opening of the channel and boosting inward currents at physiological membrane potentials. Other endogenous factors, such as phospholipase A2 products (Vanden Abeele F. et al., J. Biol.Chem. (2006) 281, 40174-40182), endocannabinoids (De Petrocellis L. et al., Exp.Cell. Res. (2007) 313, 1911-1920) and PIP2 (Rohacs T. et al., Nat. Neurosci. (2005) 8, 626-634) also participate in channel regulation.

There is a lot of direct and indirect evidence of a pivotal role of TRPM8 channel activity in diseases such as itch, irritable bowel diseases, cold induced and/or exacerbated respiratory disorders, ischemia, neurodegeneration, psychiatric disorders, stroke, urological disorders, dry eye syndrome and epiphora.

Over the last few years, several classes of non-peptide TRPM8 antagonists have been disclosed. WO 2006/040136, WO 2007/017092, WO 2007/017093, WO 2007/017094, and WO 2007/080109 describe benzyloxy derivatives as TRPM8 antagonists for the treatment of urological disorders; WO 2007/134107 describes phosphorous-bearing compounds as TRPM8 antagonists for the treatment of TRPM8-related disorders; WO 2009/012430 describes sulfonamides for the treatment of diseases associated with TRPM8; WO 2010/103381 describes the use of spirocyclic piperidine derivatives as TRPM8 modulators in prevention or treatment of TRPM8-related disorders or diseases; WO 2010/125831 describes sulfamoyl benzoic acid derivatives as modulators of the TRPM8 receptor and their use in the treatment of inflammatory, pain and urological disorders; and WO 2013/092711 describes 2-aryl oxazole and thiazole derivatives as TRPM8 receptor modulators and their use in prevention, reduction of the risk of, amelioration and/or treatment of urological-related disorders. Nonetheless, a relevant side effect of TRPM8 inhibitors which limits their clinical applicability is represented by the lowering of body temperature which is induced by these molecules (Bautista D.M. et al., Nature (2007) 448, 204-208; Knowlton W.M. et al., PLoS ONE (2011) 6, e25894; Almeida M.C. et al., J. Neurosci. (2012) 32, 2086-2099; Gavva N.R. et al., Mol. Pain. (2012) 8, 36).

The present invention is aimed at providing an effective therapeutic management of joint damage, joint degeneration and joint inflammation caused by osteoarthritis.

### SUMMARY OF THE INVENTION

The invention is directed to a TRPM8 inhibitor for use in the prevention or treatment of joint damage and/or joint degeneration and/or joint inflammation caused by osteoarthritis in a subject.

The invention is also directed to a TRPM8 inhibitor for use in the prevention or treatment of joint damage and/or joint degeneration and/or joint inflammation in a subject with osteoarthritis.

The invention is also directed to a pharmaceutical composition comprising a TRPM8 inhibitor and at least one pharmaceutically acceptable excipient or carrier for use in the prevention or treatment of joint damage and/or joint degeneration and/or joint inflammation caused by osteoarthritis in a subject.

The invention is also directed to a pharmaceutical composition comprising a TRPM8 inhibitor and at least one pharmaceutically acceptable excipient or carrier for use in the prevention or treatment of joint damage and/or joint degeneration and/or joint inflammation in a subject with osteoarthritis.

The invention is also directed to a method of preventing or treating joint damage and/or joint degeneration and/or joint inflammation caused by osteoarthritis in a subject, which comprises administering an effective amount of one or more TRPM8 inhibitors to a subject in need thereof.

The invention is also directed to a method of preventing or treating joint damage and/or joint degeneration and/or joint inflammation in a subject with osteoarthritis, which comprises administering an effective amount of one or more TRPM8 inhibitors to a subject in need thereof.

The invention is also directed to the use of a TRPM8 inhibitor in the manufacture of a medicament for the prevention or treatment of joint damage and/or joint degeneration and/or joint inflammation caused by osteoarthritis in a subject.

The invention is also directed to the use of a TRPM8 inhibitor in the manufacture of a medicament for the prevention or treatment of joint damage and/or joint degeneration and/or joint inflammation in a subject with osteoarthritis.

### BRIEF DESCRIPTION OF FIGURES

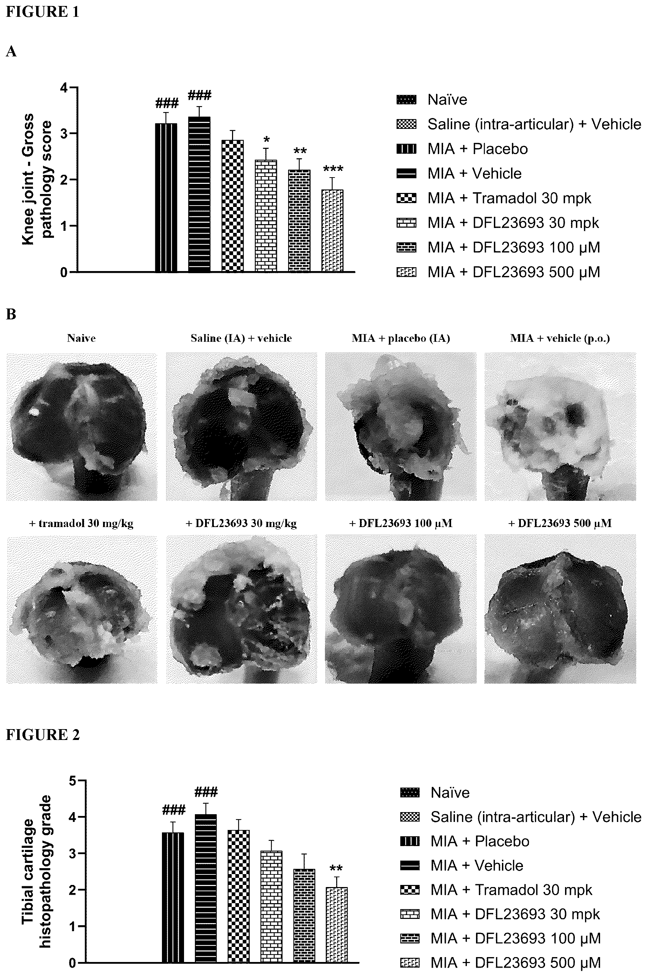

**Figure 1** shows in panel A) the effect of DFL23693 on gross pathology of knee of monosodium iodoacetate (MIA)-induced osteoarthritis. Data indicates Mean ± SEM. One-way ANOVA followed by Sidak's multiple comparisons test, n=6-14. ###p<0.001 Vs Naïve/Saline (Intra-articular) + Vehicle; *p<0.05,***p<0.001 Vs MIA + Vehicle/MIA + Placebo; and in panel B) representative images of morphology of tibial articular cartilage during MIA induced osteoarthritic changes in rat knee joint.
**Figure 2** shows the effect of DFL23693 on tibial histopathological grades of knee during monosodium iodoacetate (MIA)-induced osteoarthritis. Data indicates Mean ± SEM. One-way ANOVA followed by Sidak's multiple comparisons test, n=6-14. ###p<0.001 Vs Naïve/Saline (Intra-articular) + Vehicle;**p<0.01 Vs MIA + Placebo.

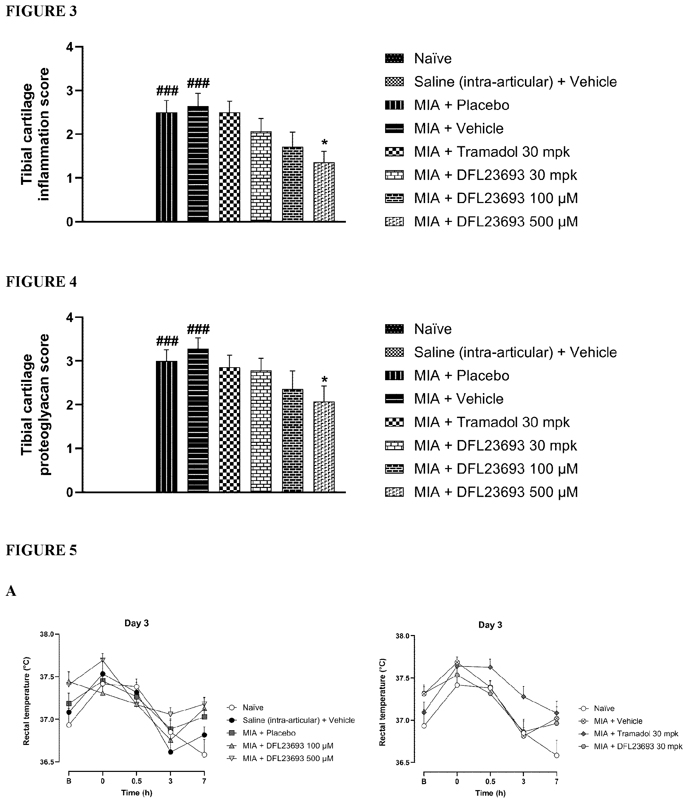

**Figure 3** shows the effect of DFL23693 on tibial cartilage inflammation score of knee in the monosodium iodoacetate (MIA)-induced osteoarthritis. Data indicates Mean ± SEM. One-way ANOVA followed by Sidak's multiple comparisons test, n=6-14. ###p<0.001 Vs Naïve/Saline (Intra-articular) + vehicle;*p<0.05 Vs MIA + Placebo.
**Figure 4** shows the effect of DFL23693 on tibial cartilage proteoglycan score of knee during monosodium iodoacetate (MIA)-induced osteoarthritis. Data indicates Mean ± SEM. One-way ANOVA followed by Sidak's multiple comparisons test, n=6-14. ###p<0.001 Vs Naïve/Saline (Intra-articular) + vehicle;*p<0.05 Vs MIA + Placebo.

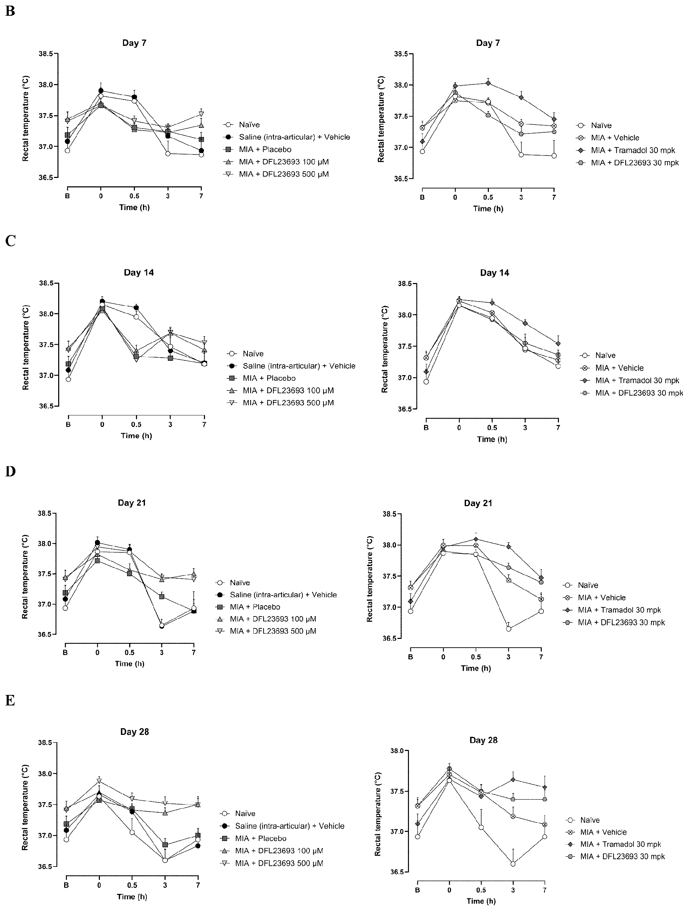

**Figure 5** shows the effect of DFL23693 on rectal temperature of monosodium iodoacetate (MIA)-induced osteoarthritic pain on days 3 (panel A), 7 (panel B), 14 (panel C), 21 (panel D) and 28 (panel E). Data indicates Mean ± SEM. Two-way ANOVA followed by Dunnett's multiple comparisons test, n=7-14.

### DETAILED DESCRIPTION OF THE INVENTION

It has been surprisingly found that inhibitors of TRPM8 are effective in the prevention and treatment of joint damage, joint degeneration and joint inflammation in subjects with osteoarthritis.

Accordingly, the invention is directed to a TRPM8 inhibitor for use in the prevention or treatment of joint damage and/or joint degeneration and/or joint inflammation caused by osteoarthritis in a subject.

According to a preferred embodiment, said TRPM8 inhibitor is for use in the prevention or treatment of joint damage or joint degeneration or joint inflammation caused by osteoarthritis in a subject.

According to a preferred embodiment, said TRPM8 inhibitor is for use in the prevention or treatment of joint damage caused by osteoarthritis in a subject.

According to a preferred embodiment, said TRPM8 inhibitor is for use in the prevention or treatment of joint degeneration caused by osteoarthritis in a subject.

According to a preferred embodiment, said TRPM8 inhibitor is for use in the prevention or treatment of joint inflammation caused by osteoarthritis in a subject.

According to a preferred embodiment, said TRPM8 inhibitor is for use in the prevention or treatment of joint damage and joint degeneration caused by osteoarthritis in a subject.

According to a preferred embodiment, said TRPM8 inhibitor is for use in the prevention or treatment of joint damage and joint inflammation caused by osteoarthritis in a subject.

According to a preferred embodiment, said TRPM8 inhibitor is for use in the prevention or treatment of joint degeneration and joint inflammation caused by osteoarthritis in a subject.

According to a preferred embodiment, said TRPM8 inhibitor is for use in the prevention or treatment of joint damage, joint degeneration and joint inflammation caused by osteoarthritis in a subject.

The invention is also directed to a TRPM8 inhibitor for use in the prevention or treatment of joint damage and/or joint degeneration and/or joint inflammation in a subject with osteoarthritis.

According to a preferred embodiment, said TRPM8 inhibitor is for use in the prevention or treatment of joint damage or joint degeneration or joint inflammation in a subject with osteoarthritis.

According to a preferred embodiment, said TRPM8 inhibitor is for use in the prevention or treatment of joint damage in a subject with osteoarthritis.

According to a preferred embodiment, said TRPM8 inhibitor is for use in the prevention or treatment of joint degeneration in a subject with osteoarthritis.

According to a preferred embodiment, said TRPM8 inhibitor is for use in the prevention or treatment of joint inflammation in a subject with osteoarthritis.

According to a preferred embodiment, said TRPM8 inhibitor is for use in the prevention or treatment of joint damage and joint degeneration in a subject with osteoarthritis.

According to a preferred embodiment, said TRPM8 inhibitor is for use in the prevention or treatment of joint damage and joint inflammation in a subject with osteoarthritis.

According to a preferred embodiment, said TRPM8 inhibitor is for use in the prevention or treatment of joint degeneration and joint inflammation in a subject with osteoarthritis.

According to a preferred embodiment, said TRPM8 inhibitor is for use in the prevention or treatment of joint damage, joint degeneration and joint inflammation in a subject with osteoarthritis.

The term "prevention" as used herein refers to the inhibition of onset of the disorder being treated or to the delay in the onset of the disorder being treated. Thus, according to a preferred embodiment, said TRPM8 inhibitor is for use in the inhibition of onset or in the delay of the onset of joint damage and/or joint degeneration and/or joint inflammation caused by osteoarthritis in a subject.

According to a further preferred embodiment, said TRPM8 inhibitor is for use in the inhibition of onset or in the delay of the onset of joint damage or joint degeneration or joint inflammation caused by osteoarthritis in a subject.

According to a further preferred embodiment, said TRPM8 inhibitor is for use in the inhibition of onset or in the delay of the onset of joint damage caused by osteoarthritis in a subject.

According to a further preferred embodiment, said TRPM8 inhibitor is for use in the inhibition of onset or in the delay of the onset of joint degeneration caused by osteoarthritis in a subject.

According to a further preferred embodiment, said TRPM8 inhibitor is for use in the inhibition of onset or in the delay of the onset of joint inflammation caused by osteoarthritis in a subject.

According to a further preferred embodiment, said TRPM8 inhibitor is for use in the inhibition of onset or in the delay of the onset of joint damage and joint degeneration and joint inflammation caused by osteoarthritis in a subject.

According to a further preferred embodiment, said TRPM8 inhibitor is for use in the inhibition of onset or in the delay of the onset of joint damage and joint degeneration caused by osteoarthritis in a subject.

According to a further preferred embodiment, said TRPM8 inhibitor is for use in the inhibition of onset or in the delay of the onset of joint damage and joint inflammation caused by osteoarthritis in a subject.

According to a further preferred embodiment, said TRPM8 inhibitor is for use in the inhibition of onset or in the delay of the onset of joint degeneration and joint inflammation caused by osteoarthritis in a subject.

According to a further preferred embodiment, said TRPM8 inhibitor is for use in the inhibition of onset or in the delay in the onset of joint damage and/or joint degeneration and/or joint inflammation in a subject with osteoarthritis.

According to a further preferred embodiment, said TRPM8 inhibitor is for use in the inhibition of onset or in the delay in the onset of joint damage or joint degeneration or joint inflammation in a subject with osteoarthritis.

According to a further preferred embodiment, said TRPM8 inhibitor is for use in the inhibition of onset or in the delay of the onset of joint damage in a subject with osteoarthritis.

According to a further preferred embodiment, said TRPM8 inhibitor is for use in the inhibition of onset or in the delay of the onset of joint degeneration in a subject with osteoarthritis.

According to a further preferred embodiment, said TRPM8 inhibitor is for use in the inhibition of onset or in the delay of the onset of joint inflammation in a subject with osteoarthritis.

According to a further preferred embodiment, said TRPM8 inhibitor is for use in the inhibition of onset or in the delay of the onset of joint damage and joint degeneration and joint inflammation in a subject with osteoarthritis.

According to a further preferred embodiment, said TRPM8 inhibitor is for use in the inhibition of onset or in the delay of the onset of joint damage and joint degeneration in a subject with osteoarthritis.

According to a further preferred embodiment, said TRPM8 inhibitor is for use in the inhibition of onset or in the delay of the onset of joint damage and joint inflammation in a subject with osteoarthritis.

According to a further preferred embodiment, said TRPM8 inhibitor is for use in the inhibition of onset or in the delay of the onset of joint degeneration and joint inflammation in a subject with osteoarthritis.

The term "treatment" as used herein refers to the eradication, mitigation or amelioration of the disorder being treated or of one or more of the symptoms associated thereof, notwithstanding the fact that the patient may still be afflicted with the underlying disorder.

Thus, according to a preferred embodiment, said TRPM8 inhibitor is for use in i) the inhibition or slowing of the progression of joint damage and/or joint degeneration and/or joint inflammation or ii) the reduction of the entity of joint damage and/or joint degeneration and/or joint inflammation caused by osteoarthritis in a subject.

According to a further preferred embodiment, said TRPM8 inhibitor is for use in the inhibition or slowing of the progression of joint damage or joint degeneration or joint inflammation caused by osteoarthritis in a subject.

According to a further preferred embodiment, said TRPM8 inhibitor is for use in the inhibition or slowing of the progression of joint damage caused by osteoarthritis in a subject.

According to a further preferred embodiment, said TRPM8 inhibitor is for use in the inhibition or slowing of the progression of joint degeneration caused by osteoarthritis in a subject.

According to a further preferred embodiment, said TRPM8 inhibitor is for use in the inhibition or slowing of the progression of joint inflammation caused by osteoarthritis in a subject.

According to a further preferred embodiment, said TRPM8 inhibitor is for use in the inhibition or slowing of the progression of joint damage and joint degeneration and joint inflammation caused by osteoarthritis in a subject.

According to a further preferred embodiment, said TRPM8 inhibitor is for use in the inhibition or slowing of the progression of joint damage and joint degeneration caused by osteoarthritis in a subject.

According to a further preferred embodiment, said TRPM8 inhibitor is for use in the inhibition or slowing of the progression of joint damage and joint inflammation caused by osteoarthritis in a subject.

According to a further preferred embodiment, said TRPM8 inhibitor is for use in the inhibition or slowing of the progression of joint degeneration and joint inflammation caused by osteoarthritis in a subject.

According to a further preferred embodiment, said TRPM8 inhibitor is for use in the reduction of the entity of joint damage or joint degeneration or joint inflammation caused by osteoarthritis in a subject.

According to a further preferred embodiment, said TRPM8 inhibitor is for use in the reduction of the entity of joint damage caused by osteoarthritis in a subject.

According to a further preferred embodiment, said TRPM8 inhibitor is for use in the reduction of the entity of joint degeneration caused by osteoarthritis in a subject.

According to a further preferred embodiment, said TRPM8 inhibitor is for use in the reduction of the entity of joint inflammation caused by osteoarthritis in a subject.

According to a further preferred embodiment, said TRPM8 inhibitor is for use in the reduction of the entity of joint damage, joint degeneration and joint inflammation caused by osteoarthritis in a subject.

According to a further preferred embodiment, said TRPM8 inhibitor is for use in the reduction of the entity of joint damage and joint degeneration caused by osteoarthritis in a subject.

According to a further preferred embodiment, said TRPM8 inhibitor is for use in the reduction of the entity of joint damage and joint inflammation caused by osteoarthritis in a subject.

According to a further preferred embodiment, said TRPM8 inhibitor is for use in the reduction of the entity of joint degeneration and joint inflammation caused by osteoarthritis in a subject.

According to a further preferred embodiment, said TRPM8 inhibitor is for use in i) the inhibition or slowing of progression of joint damage and/or joint degeneration and/or joint inflammation or ii) the reduction of the entity of joint damage and/or joint degeneration and/or joint inflammation in a subject with osteoarthritis.

According to a further preferred embodiment, said TRPM8 inhibitor is for use in the inhibition or slowing of the progression of joint damage or joint degeneration or joint inflammation in a subject with osteoarthritis.

According to a further preferred embodiment, said TRPM8 inhibitor is for use in the inhibition or slowing of the progression of joint damage in a subject with osteoarthritis.

According to a further preferred embodiment, said TRPM8 inhibitor is for use in the inhibition or slowing of the progression of joint degeneration in a subject with osteoarthritis.

According to a further preferred embodiment, said TRPM8 inhibitor is for use in the inhibition or slowing of the progression of joint inflammation in a subject with osteoarthritis.

According to a further preferred embodiment, said TRPM8 inhibitor is for use in the inhibition or slowing of the progression of joint damage and joint degeneration and joint inflammation in a subject with osteoarthritis.

According to a further preferred embodiment, said TRPM8 inhibitor is for use in the inhibition or slowing of the progression of joint damage and joint degeneration in a subject with osteoarthritis.

According to a further preferred embodiment, said TRPM8 inhibitor is for use in the inhibition or slowing of the progression of joint damage and joint inflammation in a subject with osteoarthritis.

According to a further preferred embodiment, said TRPM8 inhibitor is for use in the inhibition or slowing of the progression of joint degeneration and joint inflammation in a subject with osteoarthritis.

According to a further preferred embodiment, said TRPM8 inhibitor is for use in the reduction of the entity of joint damage or joint degeneration or joint inflammation in a subject with osteoarthritis.

According to a further preferred embodiment, said TRPM8 inhibitor is for use in the reduction of the entity of joint damage in a subject with osteoarthritis.

According to a further preferred embodiment, said TRPM8 inhibitor is for use in the reduction of the entity of joint degeneration in a subject with osteoarthritis.

According to a further preferred embodiment, said TRPM8 inhibitor is for use in the reduction of the entity of joint inflammation in a subject with osteoarthritis.

According to a further preferred embodiment, said TRPM8 inhibitor is for use in the reduction of the entity of joint damage, joint degeneration and joint inflammation in a subject with osteoarthritis.

According to a further preferred embodiment, said TRPM8 inhibitor is for use in the reduction of the entity of joint damage and joint degeneration in a subject with osteoarthritis.

According to a further preferred embodiment, said TRPM8 inhibitor is for use in the reduction of the entity of joint damage and joint inflammation in a subject with osteoarthritis.

According to a further preferred embodiment, said TRPM8 inhibitor is for use in the reduction of the entity of joint degeneration and joint inflammation in a subject with osteoarthritis.

The effect of the TRPM8 inhibitor for use according to the invention on the joint damage and/or joint degeneration and/or joint inflammation results in preservation or improvement of the functionality of the joints, and inhibition or treatment of symptoms such as stiffness and loss of mobility.

Thus, according to a further preferred embodiment, said TRPM8 inhibitor is for use in the prevention or treatment of stiffness and loss of mobility.

Preferably, according to this embodiment, said TRPM8 inhibitor is for use in the prevention or treatment of stiffness and loss of mobility caused by osteoarthritis in a subject.

Preferably, according to this embodiment, said TRPM8 inhibitor is for use in the prevention or treatment of stiffness and loss of mobility in a subject with osteoarthritis.

Preferably, according to this embodiment, said TRPM8 inhibitor is for use in i) the inhibition of onset or in the delay in of the onset of stiffness and loss of mobility, or ii) the inhibition or slowing of the progression of stiffness and loss of mobility, or iii) the reduction of the entity of stiffness and loss of mobility caused by osteoarthritis in a subject.

Preferably, according to this embodiment, said TRPM8 inhibitor is for use in i) the inhibition of onset or in the delay in of the onset of stiffness and loss of mobility, or ii) the inhibition or slowing of the progression of stiffness and loss of mobility, or iii) the reduction of the entity of stiffness and loss of mobility in a subject with osteoarthritis.

According to a preferred embodiment, said subject is a human subject.

According to a preferred embodiment, said subject is selected after a clinical evaluation of the affected joint by means of an imaging technique, preferably by means of radiography.

According to a preferred embodiment, said joint damage is joint cartilage damage.

According to a preferred embodiment, said joint degeneration is joint cartilage degeneration.

According to a preferred embodiment, said joint damage is joint bone damage. According to a preferred embodiment, said joint degeneration is joint bone degeneration.

According to a preferred embodiment, said joint damage is joint cartilage damage and joint bone damage.

According to a preferred embodiment, said joint degeneration is joint cartilage degeneration and joint bone degeneration.

Preferably, said joint bone damage is subchondral bone damage.

Preferably, said joint bone degeneration is subchondral bone degeneration.

According to a preferred embodiment, said joint damage and/or said joint degeneration includes the formation of osteophytes.

Thus, according to this embodiment, said TRPM8 inhibitor is for use in:
i) the inhibition or slowing of the formation of osteophytes, ii) the inhibition or slowing of the progression of the formation of osteophytes, or iii) the reduction of the number and/or size of osteophytes caused by osteoarthritis in a subject.

Preferably, according to this embodiment, said TRPM8 inhibitor is for use in:
i) the inhibition or slowing of the formation of osteophytes, ii) the inhibition or slowing of the progression of the formation of osteophytes, or iii) the reduction of the number and/or size of osteophytes in a subject with osteoarthritis.

According to a preferred embodiment, also in combination with any of the above embodiments, said osteoarthritis is selected from hand osteoarthritis, knee osteoarthritis, hip osteoarthritis, osteoarthritis of the lower back, osteoarthritis of the spine, ankle osteoarthritis, elbow osteoarthritis, osteoarthritis of the fingers, foot osteoarthritis, shoulder osteoarthritis, wrist osteoarthritis, first metatarsophalangeal (MTP) joint osteoarthritis, sacroiliac (SI) joint arthritis and cervical osteoarthritis. According to the most preferred embodiment, said osteoarthritis is selected from knee osteoarthritis and hand osteoarthritis, preferably it is knee osteoarthritis.

The term "TRPM8 inhibitor" in accordance with the present invention means any compound able to inhibit the biological activity of TRPM8.

Methods and tests for determining the inhibition of the biological activity of TRPM8 and for classifying a compound as "TRPM8 inhibitor" are known in the art and are described, for example, in Sui et al., Fitoterapia 145 (2020) 104631, and Chodon et al. BMC Cancer 2010, 10:212, which discloses the techniques used to analyze the expression of TRPM8 channels that can be implemented to analyze the ability of a compound to inhibit TRPM8 by inhibiting its expression.

According to a preferred embodiment, also in combination with any of the above embodiments, the TRPM8 inhibitor for use according to the invention is an antibody, preferably selected from ACC-049, ab235890, ab314169 and NBP1-97311.

According to an alternative preferred embodiment, also in combination with any of the above embodiments, the TRPM8 inhibitor for use according to the invention is a small molecule. The term "small molecule" refers to an organic compound having a molecular weight of 900 Daltons or lower.

According to a preferred embodiment, also in combination with any of the above embodiments, the TRPM8 inhibitor for use according to the invention is a TRPM8 antagonist.

To date, several TRPM8 antagonists, such as small molecules, peptides and antibodies, have been disclosed, some of which are currently undergoing clinical trials (González-Muñiz, R. et al., Int. J. Mol. Sci. 2019, 20(11), 2618; Izquierdo, C. et al., Int. J. Mol. Sci. 2021, 22(16), 8502).

According to a preferred embodiment, also in combination with any of the above embodiments, the TRPM8 antagonist for use according to the present invention is a compound of formula (I): 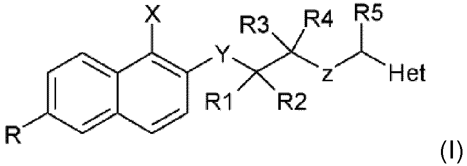
or a pharmaceutically acceptable salt thereof,
wherein
R is selected from H, Br, CN, NO₂, SO₂NH₂, SO₂NHR' and SO₂N(R')₂, where
R' is selected from linear or branched C₁-C₄ alkyl;
X is selected from F, CI, C₁-C₃ alkyl, NH₂ and OH;
Y is selected from O, CH₂, NH and SO₂;
R1 and R2, independently one from the other, are selected from H, F and linear or branched C₁-C₄ alkyl;
R₃ and R₄, independently one from the other, are selected from H and linear or branched C₁-C₄ alkyl;
Z is selected from NR6 and R6R7N⁺, where R6 and R7 independently one from the other, are selected from H and linear or branched C₁-C₄ alkyl;
R5 is a residue selected from H and linear or branched C₁-C₄ alkyl;
Het is a heteroaryl group selected from a substituted or not substituted pyrrolyl, a substituted or not substituted N-methylpyrrolyl, a substituted or not substituted thiophenyl, a substituted or not substituted furyl and a substituted or not substituted pyridinyl, and preferably it is a substituted or not substituted pyrrol-2-yl, a substituted or not substituted N- methylpyrrol-2-yl, a substituted or not substituted thiophen-2-yl, a substituted or not substituted fur-2-yl, a substituted or not substituted pyridin-2-yl.

According to preferred embodiments of the invention, also in combination one with the other, in the above compounds, independently one from the other:
R is preferably selected from H, Br and CN, and even more preferably from H and CN;
X is preferably selected from F, Cl and C₁-C₃ alkyl, more preferably from F, Cl and C₂H₅, even more preferably from F and Cl;
Y is preferably selected from -O-, CH₂, NH and SO₂, and, more preferably, from CH₂, O and SO₂;
R1 and R2, independently one from the other, are preferably selected from H, F and CH₃, more preferably from H and CH₃;
R3 and R4, independently one from the other, are preferably selected from H and CH₃;
Z is preferably selected from NR6 and R6R7N⁺ where R6 and R7, independently one from the other, are selected from H and CH₃; more preferably it is NH;
R5 is preferably selected from H and CH₃ and, more preferably it is H.

According to a further preferred embodiment of the invention, also in combination with all the above embodiments, when Het is a substituted pyrrolyl, a substituted N-methylpyrrolyl, a substituted thiophenyl or a substituted furyl, it is preferably substituted with one or more substituents selected from F, Cl, CH₃, NH₂ and OH, and more preferably from F, Cl and CH₃, even more preferably from CI and CH₃. Preferably, said substituent is in position 5.

Preferred compounds of formula (I) for use according to the present invention are listed in the following:
2-[(1-chloronaphthalen-2-yl)oxy]-N-(furan-2-ylmethyl)ethanaminium chloride;
2-[(1 -chloronaphthalen-2-yl)oxy]-N-[(5-methylfuran-2-yl)methyl]ethanaminium chloride;
N-[(5-chlorofuran-2-yl)methyl]-2-[(1-chloronaphthalen-2-yl)oxy]ethanaminium;
2-[(1-chloronaphthalen-2-yl)oxy]-N-[(5-chlorothiophen-2-yl)methyl]ethanaminium;
2-[(1-chloronaphthalen-2-yl)oxy]-N-(thiophen-2-ylmethyl)ethanaminium;
2-[(1-chloronaphthalen-2-yl)oxy]-N-(pyridin-2-ylmethyl)ethanaminium;
2-[(1-chloronaphthalen-2-yl)oxy]-N-[(1-methyl-1H-pyrrol-2-yl)methyl]ethanaminium;
1-[(1-chloronaphthalen-2-yl)oxy]-N-(furan-2-ylmethyl)-2-methylpropan-2-amine;
1-[(1-chloronaphthalen-2-yl)oxy]-2-methyl-N-[(5-methylfuran-2-yl)methyl]propan-2- amine;
N-[(5-chlorofuran-2-yl)methyl]-1-[(1-chloronaphthalen-2-yl)oxy]-2-methylpropan-2- amine;
1-[(1-chloronaphthalen-2-yl)oxy]-N-[(5-chlorothiophen-2-yl)methyl]-2-methylpropan-2- amine;
1-[(1-chloronaphthalen-2-yl)oxy]-2-methyl-N-(thiophen-2-ylmethyl)propan-2-amin;
1-[(1-chloronaphthalen-2-yl)oxy]-2-methyl-N-(pyridin-2-ylmethyl)propan-2-amine;
1-[(1-chloronaphthalen-2-yl)oxy]-2-methyl-N-[(1-methyl-1H-pyrrol-2-yl)methyl]propan-2-am ine;
2-[(1-chloronaphthalen-2-yl)oxy]-N-(furan-2-ylmethyl)propan-1-am ine;
2-[(1-chloronaphthalen-2-yl)oxy]-N-[(5-methylfuran-2-yl)methyl]propan-1-amine;
N-[(5-chlorofuran-2-yl)methyl]-2-[(1-chloronaphthalen-2-yl)oxy]propan-1-amine;
2-[(1-chloronaphthalen-2-yl)oxy]-N-[(5-chlorothiophen-2-yl)methyl]propan-1-amine;
2-[(1-chloronaphthalen-2-yl)oxy]-N-(thiophen-2-ylmethyl)propan-1-amine;
1-[(1-chloronaphthalen-2-yl)oxy]-N-(furan-2-ylmethyl)propan-2-amine;
1-[(1-chloronaphthalen-2-yl)oxy]-N-[(5-methylfuran-2-yl)methyl]propan-2-amine;
N-[(5-chlorofuran-2-yl)methyl]-1-[(1-chloronaphthalen-2-yl)oxy]propan-2-amine;
1-[(1-chloronaphthalen-2-yl)oxy]-N-[(5-chlorothiophen-2-yl)methyl]propan-2-amine;
1-[(1-chloronaphthalen-2-yl)oxy]-N-(thiophen-2-ylmethyl)propan-2-amine;
2-[(1 -chloronaphthalen-2-yl)sulfonyl]-N-(furan-2-ylmethyl)ethanamine;
2-[(1-chloronaphthalen-2-yl)sulfonyl]-N-[(5-methylfuran-2-yl)methyl]ethanamine;
N-[(5-chlorofuran-2-yl)methyl]-2-[(1-chloronaphthalen-2-yl)sulfonyl]ethanamine;
2-[(1-chloronaphthalen-2-yl)sulfonyl]-N-[(5-chlorothiophen-2-yl)methyl]ethanamine;
2-[(1 -chloronaphthalen-2-yl)sulfonyl]-N-(thiophen-2-ylmethyl)ethanamine;
2-[(1 -chloronaphthalen-2-yl)sulfonyl]-N-(pyridin-2-ylmethyl)ethanamine;
2-[(1-chloronaphthalen-2-yl)sulfonyl]-N-[(1-methyl-1H-pyrrol-2-yl)methyl]ethanamine;
3-(1-chloronaphthalen-2-yl)-N-(furan-2-ylmethyl)propan-1-amine;
3-(1-chloronaphthalen-2-yl)-N-[(5-methylfuran-2-yl)methyl]propan-1-amine;
N-[(5-chlorofuran-2-yl)methyl]-3-(1-chloronaphthalen-2-yl)propan-1-am ine;
3-(1-chloronaphthalen-2-yl)-N-[(5-chlorothiophen-2-yl)methyl]propan-1-amine;
3-(1-chloronaphthalen-2-yl)-N-(thiophen-2-ylmethyl)propan-1-amine;
3-(1-chloronaphthalen-2-yl)-N-(pyridin-2-ylmethyl)propan-1-amine;
2-[(1 -chloronaphthalen-2-yl)oxy]-N-(furan-2-ylmethyl)-N-methylethanamine;
2-[(1-chloronaphthalen-2-yl)oxy]-N-(furan-2-ylmethyl)-N,N-dimethylethanaminium iodide;
N-{2-[(1-chloronaphthalen-2-yl)oxy]ethyl}-1-(5-methylfuran-2-yl)ethanamine;
N-(1-chloronaphthalen-2-yl)-N'-(furan-2-ylmethyl)ethane-1,2-diamine;
N-(I-chloronaphthalen-2-yl)-N'-[(5-methylfuran-2-yl)methyl]ethane-1,2-diamine;
N-[(5-chloromran-2-yl)methyl]-N'-(I-chloronaphthalen-2-yl)ethane-1,2-diamine;
N-(1-chloronaphthalen-2-yl)-N'-[(5-chlorothiophen-2-yl)methyl]ethane-1,2-diamine;
N-(1-chloronaphthalen-2-yl)-N'-(thiophen-2-ylmethyl)ethane-1,2-diamine; N-(1-chloronaphthalen-2-yl)-N'-(pyridin-2-ylmethyl)ethane-1,2-diamine;
N-(1-chloronaphthalen-2-yl)-N'-[(1-methyl-1H-pyrrol-2-yl)methyl]ethane-1,2-diamine;
2-[(1 -fluoronaphthalen-2-yl)oxy]-N-(furan-2-ylmethyl)ethanamine;
2-[(1 -fluoronaphthalen-2-yl)oxy]-N-[(5-methylfuran-2-yl)methyl]ethanamine;
N-[(5-chlorofuran-2-yl)methyl]-2-[(1-fluoronaphthalen-2-yl)oxy]ethanamine;
N-[(5-chlorothiophen-2-yl)methyl]-2-[(1-fluoronaphthalen-2-yl)oxy]ethanamine;
2-[(1 -fluoronaphthalen-2-yl)oxy]-N-(thiophen-2-ylmethyl)ethanamine;
2-[(1 -fluoronaphthalen-2-yl)oxy]-N-(pyridin-2-ylmethyl)ethanamine;
2-[(1-fluoronaphthalen-2-yl)oxy]-N-[(1-methyl-1H-pyrrol-2-yl)methyl]ethanamine;
5-chloro-6-{2-[(pyridin-2-ylmethyl)amino]ethoxy}naphthalene-2 -carbonitrile;
5-chloro-6-{2-[(furan-2-ylmethyl)amino]ethoxy}naphthalene-2-carbonitrile;
5-chloro-6-(2-{[(5-methylfuran-2-yl)methyl]amino}ethoxy)naphthalene-2-carbonitrile;
5-chloro-6-(2-{[(5-chlorofuran-2-yl)methyl]am ino}ethoxy)naphthalene-2-carbonitrile;
5-chloro-6-(2-{[(5-chlorothiophen-2-yl)methyl]amino}ethoxy)naphthalene-2-carbonitrile;
5-chloro-6-{2-[(thiophen-2-ylmethyl)amino]ethoxy}naphthalene-2-carbonitrile;
2-[(1 -ethylnaphthalen-2-yl)oxy]-N-(furan-2-ylmethyl)ethanamine;
2,2-difluoro-2-[(1-fluoronaphthalen-2-yl)oxy]-N-(furan-2-ylmethyl)ethanamine; and
2-[(6-bromo-1-fluoronaphthalen-2-yl)oxy]-N-(furan-2-ylmethyl)ethanamine.

Particularly preferred compounds of formula (I) for use according to the present invention are:
2-[(1-chloronaphthalen-2-yl)oxy]-N-(furan-2-ylmethyl)ethanaminium chloride;
2-[(1-chloronaphthalen-2-yl)oxy]-N-(thiophen-2-ylmethyl)ethanaminium;
2-[(1-chloronaphthalen-2-yl)oxy]-N-(pyridin-2-ylmethyl)ethanam inium;
1-[(1-chloronaphthalen-2-yl)oxy]-N-(furan-2-ylmethyl)-2-methylpropan-2-amine;
1-[(1-chloronaphthalen-2-yl)oxy]-2-methyl-N-(pyridin-2-ylmethyl)propan-2-amine;
1-[(1-chloronaphthalen-2-yl)oxy]-2-methyl-N-[(1-methyl-1H-pyrrol-2-yl)methyl]propan-2-am ine;
2- [(1-chloronaphthalen-2-yl)sulfonyl]-N-(furan-2-ylmethyl)ethanamine;
3-(1-chloronaphthalen-2-yl)-N-(furan-2-ylmethyl)propan-1-amine;
3-(1-chloronaphthalen-2-yl)-N-[(5-methylfuran-2-yl)methyl]propan-1-amine;
N-[(5-chlorofuran-2-yl)methyl]-3-(1-chloronaphthalen-2-yl)propan-1-amine;
3-(1-chloronaphthalen-2-yl)-N-[(5-chlorothiophen-2-yl)methyl]propan-1-amine;
2-[(1-fluoronaphthalen-2-yl)oxy]-N-(furan-2-ylmethyl)ethanamine;
2-[(1-fluoronaphthalen-2-yl)oxy]-N-(thiophen-2-ylmethyl)ethanamine;
2-[(1-fluoronaphthalen-2-yl)oxy]-N-(pyridin-2-ylmethyl)ethanamine;
5-chloro-6-{2-[(pyridin-2-ylmethyl)amino]ethoxy}naphthalene-2-carbonitrile; and
5-chloro-6-{2-[(thiophen-2-ylmethyl)amino]ethoxy}naphthalene-2-carbonitrile.

Compounds of formula (I) are disclosed in WO2012/101244A1, which also discloses their methods of synthesis and their inhibitory activity towards TRPM8.

According to a preferred embodiment, also in combination with any of the above embodiments, said TRPM8 antagonist for use according to the present invention is a compound of formula (II) 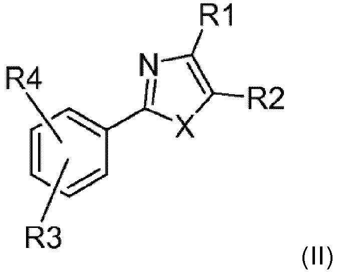 or a pharmaceutically acceptable salt thereof,
wherein
X is selected from S and O;
R1 is selected from the group consisting of:
   - OR5 wherein R5 is selected from H; C₁-C₄ alkyl, trifluoromethanesulfonyl, benzyl, (trifluoromethyl)benzyl, (halo)benzyl, (trifluoromethyl)benzoyl, N-benzylcarbamoyl, cyclohexyloxyacetoyl substituted with at least one C₁-C₃ alkyl group, (C₁-C₃ alkoxy)methyl, C₁-C₃ alkanoyl and CH₂CH₂NHR6, wherein R6 is selected from H and (furan-2-yl)methyl; and
   - NHR7 wherein R7 is selected from H, tert-butoxycarbonyl, C₁-C₃ alkanoyl, (4-trifluoromethyl)benzoyl, N-phenylaminoacarbonyl, CH₂R8, wherein R8 is selected from phenyl, benzo[d][1,3] dioxole, pyridin-3-yl, (pyrrolidin-1-yl) methyl, -CH₂NHR9 wherein R9 is selected from H, C₁-C₃ alkyl and cycloalkyl; R2 is selected from the group consisting of
   - COOR10 wherein R10 is selected from H, C₁-C₃ alkyl and cyclohexyl, optionally substituted with at least one C₁-C₃ alkyl group;
   - OH; -CONH₂; CN; -tetrazol-5-yl, 1-(C₁-C₃ alkyl)tetrazol-5-yl, 2-(C₁-C₃ alkyl)tetrazol-5-yl, 5-(C₁-C₃ alkyl)1,2,4 triazol-3-yl, 5-(C₁-C₃ alkyl)1,2,4-oxadiazol-3yl, 5-(C₁-C₃ alkyl) 1,3,4-oxadiazol-2-yl;
R3 is selected from F and H,
R4 is selected from H; CH₃; halogen; dimethylamino; pyridin-4yl; phenyl; 2- or 4- (halo)phenyl; 2- or 4- (trifluoromethyl)phenyl; 2- and/or 4-halobenzyloxy.

According to a preferred embodiment, in said compounds of formula (II), R5 may be selected from H, C₁-C₄ alkyl, trifluoromethanesulfonyl, benzyl, (trifluoromethyl)benzyl, (chloro)benzyl, (trifluoromethyl)benzoyl, N-benzylcarbamoyl, cyclohexyloxyacetoyl substituted with at least one C₁-C₃ alkyl group, (methoxy)methyl, propanoyl and CH₂CH₂NHR6 wherein R6 is as above.

Particularly preferred among compounds of formula (II) are compounds wherein R5 is selected from H, methyl, isobutyl, trifluoromethanesulfonyl, benzyl, 4-(trifluoromethyl)benzyl, (chloro)benzyl, 4-(trifluoromethyl)benzoyl, N-benzylcarbamoyl, 2-isopropyl-5-methylcyclohexyloxyacetoyl, (methoxy)methyl, propanoyl and CH₂CH₂NHR6 wherein R6 is as above.

According to a further preferred embodiment, also in combination with any of the preceding embodiment, in said compounds of formula (II) R7 may be selected from H, tert-butoxycarbonyl, acetyl, 4-(trifluoromethyl)benzoyl, N-phenylaminoacarbonyl, CH₂R8, wherein R8 is selected from phenyl, benzo[d][1 ,3] dioxole, pyridin-3-yl, (pyrrolidin-1-yl)methyl, -CH₂NHR9 wherein R9 is selected from H, C₁-C₃ alkyl and cyclopentyl.

According to a further preferred embodiment, also in combination with any one of the preceding embodiments, in said compounds of formula (II) R10 may be selected from H, C₁-C₃ alkyl and 2-isopropyl-5-cyclohexyl.

According to a further preferred embodiment, also in combination with any one of the preceding embodiments, in said compounds of formula (II) R4 may be selected from H, CH₃, F, CI, dimethylamino, preferably in position para, pyridin-4yl, phenyl, 2-F-phenyl, 2-trifluoromethylphenyl and 2- or 4-halobenzyloxy, wherein said halo is preferably F or CI.

According to a further preferred embodiment, in said compounds of formula (II)
X is selected from S and O;
R1 is selected from the group consisting of:
   - OR5 wherein R5 is selected from H, C₁-C₄ alkyl, trifluoromethanesulfonyl, benzyl, (trifluoromethyl)benzyl, (chloro)benzyl, (trifluoromethyl)benzoyl, N-benzylcarbamoyl, cyclohexyloxyacetoyl substituted with at least one C₁-C₃ alkyl group, (methoxy)methyl, propanoyl and -CH₂CH₂NHR6, wherein R6 is selected from H and (furan-2-yl)methyl
   - NHR7 wherein R7 is selected from H, tert-butoxycarbonyl, acetyl, (4-trifluoromethyl)benzoyl, N-phenylaminocarbonyl, CH₂R8, wherein R8 is selected from phenyl, benzo[d][1,3] dioxole, pyridin-3-yl, (pyrrolidin-1-yl)methyl, -CH₂NHR9 wherein R9 is selected from H, C₁-C₃ alkyl and cyclopentyl
R2 is selected from the group consisting of:
   - COOR10 wherein R10 is selected from H, C₁-C₃ alkyl and 2-isopropyl-5-methylcyclohexyloxycarbonyl,
   - OH; -CONH₂; CN; tetrazol-5-yl or 1-(C₁-C₃ alkyl)tetrazol-5-yl; 2-(C₁-C₃ alkyl)tetrazol-5-yl; 5-( C₁-C₃ alkyl)1,2,4 triazol-3-yl;- 5-(C₁-C₃ alkyl) 1,2,4-oxadiazol-3yl; -5-(C₁-C₃ alkyl) 1,3,4-oxadiazol-2-yl;
R3 is selected from F and H,
R4 is selected from H, F, Cl, dimethylamino, preferably in position para, pyridin-4yl, phenyl, 2-F-phenyl, 2-trifluoromethylphenyl, 2- and/or 4-F-benzyloxy.

Particularly preferred compounds of formula (II) for use according to the invention are compounds of formula (II) wherein R1 is selected from:
- OR5, wherein R5 is selected from H, benzyl, (chloro)benzyl, (trifluoromethyl)benzoyl, CH₂-CH₂NH₂; and
- NHCH₂CH₂R9 wherein R9 is selected from H and C₁-C₃ alkyl.

Particularly preferred are also compounds of formula (II) wherein R2 is selected from COOR10 wherein R10 is selected from H and C₁-C₃ alkyl.

Particularly preferred are also compounds of formula (II) wherein R3 is H.

Particularly preferred among the above compounds are those compounds of formula (II) wherein:
R1 is selected from:
OR5, wherein R5 is selected from H, benzyl, (chloro)benzyl, (trifluoromethyl)benzoyl; and
CH₂-CH₂NH₂; and
NHCH₂CH₂R9 wherein R9 is selected from C₁-C₃ alkyl and H;
R2 is COOR10 wherein R10 is selected from H, C₁-C₃ alkyl
R3 is H.

According to a preferred embodiment, also in combination with any preceding embodiment, when X is S, in the above compounds of formula (II) when R1 is OH and R2 is COOH, R4 is different from Cl in meta position on the aromatic ring.

According to another preferred embodiment, also in combination with any preceding embodiment, when R1 is OH and R2 is COOH or COOEt, R3 and R4 are not H at the same time.

According to a further preferred embodiment, also in combination with any preceding embodiments, in said compounds of formula (II) when R3 is F, R3 is in position ortho of the aromatic ring and R4 is F in position para of the aromatic ring, and when R3 is H, R4 is in position para or meta on the aromatic ring.

According to a further preferred embodiment, the compound of formula (II) for use according to the present invention is selected from:
2-(4-chlorophenyl)-4-hydroxy-1,3-thiazole-5-carboxylic acid;
4-hydroxy-2-(4-methylphenyl)-1,3-thiazole-5-carboxylic acid;
2-(3-fluorophenyl)-4-hydroxy-1,3-thiazole-5-carboxylic acid;
2-(4-fluorophenyl)-4-hydroxy-1,3-thiazole-5-carboxylic acid;
methyl 4-hydroxy-2-phenyl-1,3-thiazole-5-carboxylate;
methyl 2-(2,4-difluorophenyl)-4-hydroxy-1,3-thiazole-5-carboxylate;
ethyl 4-hydroxy-2-phenyl-1,3-thiazole-5-carboxylate;
ethyl 2-(4-chlorophenyl)-4-hydroxy-1,3-thiazole-5-carboxylate;
ethyl 4-hydroxy-2-(4-methylphenyl)-1,3-thiazole-5-carboxylate;
ethyl 2-(3-fluorophenyl)-4-hydroxy-1,3-thiazole-5-carboxylate;
ethyl 2-(4-fluorophenyl)-4-hydroxy-1,3-thiazole-5-carboxylate;
ethyl 4-hydroxy-2-(pyridin-4-yl)-1,3-thiazole-5-carboxylate;
ethyl 2-[4-(dimethylamino)phenyl]-4-hydroxy-1,3-thiazole-5-carboxylate;
ethyl 2-(3-chlorophenyl)-4-hydroxy-1,3-thiazole-5-carboxylate;
ethyl 4-hydroxy-2-[2'-(trifluoromethyl)biphenyl-3-yl]-1,3-thiazole-5-carboxylate;
ethyl 2-(2'-fluorobiphenyl-3-yl)-4-hydroxy-1,3-thiazole-5-carboxylate;
ethyl 4-hydroxy-2-[2'-(trifluoromethyl)biphenyl-4-yl]-1,3-thiazole-5-carboxylate;
ethyl 2-(2'-fluorobiphenyl-4-yl)-4-hydroxy-1,3-thiazole-5-carboxylate;
ethyl 2-{4-[(2-fluorobenzyl)oxy]phenyl}-4-hydroxy-1,3-thiazole-5-carboxylate;
ethyl 2-{4-[(4-fluorobenzyl)oxy]phenyl}-4-hydroxy-1,3-thiazole-5-carboxylate;
ethyl 2-(4-fluorophenyl)-4-{[(trifluoromethyl)sulfonyl]oxy}-1,3-thiazole-5-carboxylate;
ethyl 4-methoxy-2-(4-methylphenyl)-1,3-thiazole-5-carboxylate;
ethyl 2-(4-methylphenyl)-4-(2-methylpropoxy)-1,3-thiazole-5-carboxylate;
ethyl 4-(benzyloxy)-2-phenyl-1,3-thiazole-5-carboxylate;
ethyl 4-[(3-chlorobenzyl)oxy]-2-(4-chlorophenyl)-1,3-thiazole-5-carboxylate;
ethyl 4-[(3-chlorobenzyl)oxy]-2-(3-fluorophenyl)-1,3-thiazole-5-carboxylate;
ethyl 4-[(4-chlorobenzyl)oxy]-2-phenyl-1,3-thiazole-5-carboxylate;
ethyl 4-[(4-chlorobenzyl)oxy]-2-(3-chlorophenyl)-1,3-thiazole-5-carboxylate;
ethyl 4-[(4-chlorobenzyl)oxy]-2-(4-methylphenyl)-1,3-thiazole-5-carboxylate;
ethyl 4-[(4-chlorobenzyl)oxy]-2-(3-fluorophenyl)-1,3-thiazole-5-carboxylate;
ethyl 4-[(2-chlorobenzyl)oxy]-2-phenyl-1,3-thiazole-5-carboxylate;
ethyl 4-[(2-chlorobenzyl)oxy]-2-(4-fluorophenyl)-1,3-thiazole-5-carboxylate;
ethyl 4-[(2-chlorobenzyl)oxy]-2-(4-chlorophenyl)-1,3-thiazole-5-carboxylate;
ethyl 4-[(2-chlorobenzyl)oxy]-2-(3-chlorophenyl)-1,3-thiazole-5-carboxylate;
ethyl 4-[(2-chlorobenzyl)oxy]-2-(4-methylphenyl)-1,3-thiazole-5-carboxylate;
ethyl 2-phenyl-4-{[4-(trifluoromethyl)benzoyl]oxy}-1,3-thiazole-5-carboxylate;
ethyl 2-(3-fluorophenyl)-4-{[4-(trifluoromethyl)benzoyl]oxy}-1,3-thiazole-5-carboxylate;
ethyl 2-(4-methylphenyl)-4-{[4-(trifluoromethyl)benzoyl]oxy}-1,3-thiazole-5-carboxylate;
ethyl 4-(2-((1R,2S,5R)-2-isopropyl-5-methylcyclohexyloxy)acetoyloxy)-2-(4-methylphenyl)-1,3-thiazole-5-carboxylate;
ethyl 4-[(benzylcarbamoyl)oxy]-2-(4-chlorophenyl)-1,3-thiazole-5-carboxylate;
ethyl 4-(2-aminoethoxy)-2-(4-chlorophenyl)-1,3-thiazole-5-carboxylate;
ethyl 2-(4-chlorophenyl)-4-{2-[(furan-2-ylmethyl)amino]ethoxy}-1,3-thiazole-5-carboxylate;
4-[(4-chlorobenzyl)oxy]-2-(4-methylphenyl)-1,3-thiazole-5-carboxylic acid;
4-[(4-chlorobenzyl)oxy]-2-phenyl-1,3-thiazole-5-carboxylic acid;
4-[(4-chlorobenzyl)oxy]-2-(4-chlorophenyl)-1,3-thiazole-5-carboxylic acid;
4-[(4-chlorobenzyl)oxy]-2-(3-chlorophenyl)-1,3-thiazole-5-carboxylic acid;
4-(benzyloxy)-2-phenyl-1,3-thiazole-5-carboxylic acid;
4-[(3-chlorobenzyl)oxy]-2-(3-fluorophenyl)-1,3-thiazole-5-carboxylic acid;
4-[(2-chlorobenzyl)oxy]-2-phenyl-1,3-thiazole-5-carboxylic acid;
4-[(2-chlorobenzyl)oxy]-2-(4-fluorophenyl)-1,3-thiazole-5-carboxylic acid;
4-[(2-chlorobenzyl)oxy]-2-(4-chlorophenyl)-1,3-thiazole-5-carboxylic acid;
4-[(2-chlorobenzyl)oxy]-2-(3-chlorophenyl)-1,3-thiazole-5-carboxylic acid;
4-[(2-chlorobenzyl)oxy]-2-(4-methylphenyl)-1,3-thiazole-5-carboxylic acid;
4-[(2-chlorobenzyl)oxy]-2-(3-fluorophenyl)-1,3-thiazole-5-carboxylic acid;
2-phenyl-4-{[4-(trifluoromethyl)benzyl]oxy}-1,3-thiazole-5-carboxylic acid;
2-(3-fluorophenyl)-4-{[4-(trifluoromethyl)benzyl]oxy}-1,3-thiazole-5-carboxylic acid;
2-phenyl-4-{[4-(trifluoromethyl)benzoyl]oxy}-1,3-thiazole-5-carboxylic acid;
2-(3-fluorophenyl)-4-{[4-(trifluoromethyl)benzoyl]oxy}-1,3-thiazole-5-carboxylic acid;
2-(4-methylphenyl)-4-{[4-(trifluoromethyl)benzoyl]oxy}-1,3-thiazole-5-carboxylic acid;
4-methoxy-2-(4-methylphenyl)-1,3-thiazole-5-carboxylic acid;
2-(4-methylphenyl)-4-(2-methylpropoxy)-1,3-thiazole-5-carboxylic acid;
ethyl 4-[(tert-butoxycarbonyl)amino]-2-(4-fluorophenyl)-1,3-thiazole-5-carboxylate;
ethyl 4-amino-2-(4-fluorophenyl)-1,3-thiazole-5-carboxylate hydrochloride;
ethyl 4-(acetylamino)-2-(4-methylphenyl)-1,3-thiazole-5-carboxylate;
ethyl 2-(4-methylphenyl)-4-{[4-(trifluoromethyl)benzoyl]amino}-1,3-thiazole-5-carboxylate;
ethyl 2-(4-methylphenyl)-4-[(phenylcarbamoyl)amino]-1,3-thiazole-5-carboxylate;
ethyl 4-[(2-aminoethyl)amino]-2-(4-methylphenyl)-1, 3-thiazole-5-carboxylate; ethyl 2-(4-chlorophenyl)-4-{[2-(methylamino)ethyl]amino}-1,3-thiazole-5-carboxylate;
ethyl 2-(4-chlorophenyl)-4-{[2-(propylamino)ethyl]amino}-1,3-thiazole-5-carboxylate;
ethyl 4-[(2-aminoethyl)amino]-2-(4-chlorophenyl)-1,3-thiazole-5-carboxylate;
ethyl 4-{[2-(methylamino)ethyl]amino}-2-(4-methylphenyl)-1,3-thiazole-5-carboxylate;
ethyl 4-[(2-aminoethyl)amino]-2-[2'-(trifluoromethyl)biphenyl-4-yl]-1,3-thiazole-5-carboxylate;
ethyl 4-[(2-aminoethyl)amino]-2-[2'-(trifluoromethyl)biphenyl-3-yl]-1,3-thiazole-5-carboxylate;
ethyl 2-(4-chlorophenyl)-4-{[2-(cyclopentylamino)ethyl]amino}-1,3-thiazole-5-carboxylate;
ethyl 2-phenyl-4-{[2-(pyrrolidin-1-yl)ethyl]amino}-1,3-thiazole-5-carboxylate;
ethyl 4-(benzylamino)-2-(3-fluorophenyl)-1,3-thiazole-5-carboxylate;
ethyl 4-[(1,3-benzodioxol-5-ylmethyl)amino]-2-(3-fluorophenyl)-1,3-thiazole-5-carboxylate;
ethyl 2-(3-fluorophenyl)-4-[(pyridin-3-ylmethyl)amino]-1,3-thiazole-5-carboxylate;
4-[(2-aminoethyl)amino]-2-(4-methylphenyl)-1,3-thiazole-5-carboxylic acid;
4-{[2-(methylamino)ethyl]amino}-2-(4-methylphenyl)-1,3-thiazole-5-carboxylic acid;
4-[(2-aminoethyl)amino]-2-(3-fluorophenyl)-1,3-thiazole-5-carboxylic acid;
sodium 4-[(3-chlorobenzyl)oxy]-2-(3-fluorophenyl)-1,3-thiazole-5-carboxylate;
sodium 4-[(4-chlorobenzyl)oxy]-2-(4-methylphenyl)-1,3-thiazole-5-carboxylate;
sodium 4-(4-chlorobenzyloxy)-2-(4-chlorophenyl)-1,3-thiazole-5-carboxylate;
sodium 4-(2-chlorobenzyloxy)-2-(4-chlorophenyl)-1,3-thiazole-5-carboxylate;
sodium 4-(2-chlorobenzyloxy)-2-(4-methylphenyl)-1,3-thiazole-5-carboxylate;
sodium 4-(2-chlorobenzyloxy)-2-(3-fluorophenyl)-1,3-thiazole-5-carboxylate;
sodium 4-(4-chlorobenzyloxy)-2-(3-fluorophenyl)-1,3-thiazole-5-carboxylate;
(1R,2S,5R)-2-isopropyl-5-methylcyclohexyl-4-(benzyloxy)-2-(4-methylphenyl)-1,3-thiazole-5-carboxylate;
(1R,2S,5R)-2-isopropyl-5-methylcyclohexyl-4-hydroxy-2-(4-methylphenyl)-1,3-thiazole-5-carboxylate;
ethyl 2-(4-chlorophenyl)-4-(methoxymethoxy)-1,3-thiazole-5-carboxylate;
2-(4-chlorophenyl)-4-(methoxymethoxy)-1,3-thiazole-5-carboxylic acid;
2-(4-chlorophenyl)-4-(methoxymethoxy)-1,3-thiazole-5-carboxamide;
2-(4-chlorophenyl)-4-(methoxymethoxy)-1,3-thiazole-5-carbonitrile;
2-(4-chlorophenyl)-5-(1H-tetrazol-5-yl)-1,3-thiazol-4-ol;
2-(4-chlorophenyl)-5-(1-methyl-1H-tetrazol-5-yl)-1,3-thiazol-4-ol;
2-(3-fluorophenyl)-5-(1-methyl-1H-tetrazol-5-yl)-1,3-thiazol-4-ol;
2-(4-chlorophenyl)-5-(5-methyl-4H-1,2,4-triazol-3-yl)-1,3-thiazol-4-ol;
2-(3-fluorophenyl)-5-(5-methyl-4H-1,2,4-triazol-3-yl)-1,3-thiazol-4-ol;
2-(4-chlorophenyl)-5-(5-methyl-1,2,4-oxadiazol-3-yl)-1,3-thiazol-4-ol;
2-(3-fluorophenyl)-5-(5-methyl-1,2,4-oxadiazol-3-yl)-1,3-thiazol-4-ol;
3-{4-[(4-chlorobenzyl)oxy]-2-(3-fluorophenyl)-1,3-thiazol-5-yl}-5-methyl-1,2,4-oxadiazole;
2-(4-chlorophenyl)-5-(5-methyl-1,3,4-oxadiazol-2-yl)-1,3-thiazol-4-ol;
2-(3-fluorophenyl)-5-(5-methyl-1,3,4-oxadiazol-2-yl)-1,3-thiazol-4-ol;
ethyl 4-hydroxy-2-phenyl-1,3-oxazole-5-carboxylate;
ethyl 2-(3-fluorophenyl)-4-hydroxy-1,3-oxazole-5-carboxylate;
ethyl 4-hydroxy-2-(4-methylphenyl)-1,3-oxazole-5-carboxylate;
ethyl 4-[(4-chlorobenzyl)oxy]-2-phenyl-1,3-oxazole-5-carboxylate;
ethyl 4-[(4-chlorobenzyl)oxy]-2-(3-fluorophenyl)-1,3-oxazole-5-carboxylate;
ethyl 4-[(4-chlorobenzyl)oxy]-2-(4-methylphenyl)-1,3-oxazole-5-carboxylate;
ethyl 2-phenyl-4-{[4-(trifluoromethyl)benzoyl]oxy}-1,3-oxazole-5-carboxylate;
4-[(4-chlorobenzyl)oxy]-2-phenyl-1,3-oxazole-5-carboxylic acid;
4-[(4-chlorobenzyl)oxy]-2-(3-fluorophenyl)-1,3-oxazole-5-carboxylic acid;
4-[(4-chlorobenzyl)oxy]-2-(4-methylphenyl)-1,3-oxazole-5-carboxylic acid;
2-(3-fluorophenyl)-5-(5-methyl-1,2,4-oxadiazol-3-yl)-1,3-oxazol-4-ol;
3-{4-[(4-chlorobenzyl)oxy]-2-(3-fluorophenyl)-1,3-oxazol-5-yl}-5-methyl-1,2,4-oxadiazole;
ethyl 2-(3-fluorophenyl)-5-hydroxy-1,3-thiazole-4-carboxylate; and
2-(3-fluorophenyl)-5-(2-ethyl-2H-tetrazol-5-yl)-1,3-thiazol-4-ol, also known as DFL23448.

Compounds of formula (II) are disclosed in WO2013/092711A1, which also discloses their method of synthesis and their inhibitory activity towards TRPM8.

According to a further preferred embodiment, also in combination with any of the above embodiments, said TRPM8 antagonist for use according to the invention is a compound of formula (III) 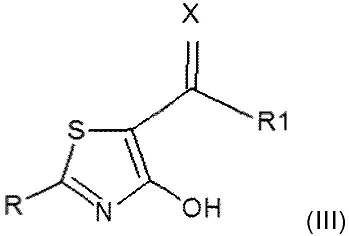
or a pharmaceutically acceptable salt thereof,
wherein
X is oxygen, sulphur, NH, NOH, or NOMe;
R is a group selected from aryl and heteroaryl, optionally substituted by one or more substituents selected from
   - hydrogen,
   - halogen,
   - CF₃,
   - linear or branched C₁-C₆ alkyl,
   - OR5 and
   - NR6R7, wherein R5, R6 and R7 are independently hydrogen or linear or branched C₁-C₆ alkyl;
R1 is a group selected from
   - linear or branched C₁-C₆ alkyl,
   - (CH₂)ₘ-OR2, wherein m is an integer between 1 and 3 and R2 is selected from hydrogen and linear C₁-C₃ alkyl,
   - C₃-C₆ cycloalkyl, and
   - N(R3)OR4, wherein R3 and R4 are independently hydrogen or linear or branched C₁-C₃ alkyl.

According to a first preferred embodiment in said compounds of formula (III) R1 is selected from:
- linear or branched C₁-C₆ alkyl,
- (CH₂)ₘ-OR2 wherein m is 1 and R2 is linear C₁-C₃ alkyl,
- C₃-C₆ cycloalkyl, or
- N(R3)OR4, wherein R3 and R4 are as defined above.

Particularly preferred compounds according to this embodiment are compounds of formula (III) wherein R1 is
- linear or branched C₁-C₆ alkyl,
- (CH₂)ₘ-OR2 wherein m is 1 and R2 is CH₃,
- cyclopropyl,
- or
- N(R3)OR4, wherein R3 and R4 are independently C₁-C₃ alkyl, preferably CH₃.

According to a second preferred embodiment, also in combination with the preceding embodiment, in the above compounds of formula (III), R1 is not methyl. Particularly preferred compounds according of this embodiment are compounds wherein R1 is selected from the group consisting of ethyl, isopropyl, isobutyl, CH₂OCH₃, cyclopropyl and N(CH₃)OCH₃.

According to a third preferred embodiment, also in combination with the first embodiment mentioned above, R1 is selected from the group consisting of methyl, ethyl, isopropyl, isobutyl, CH₂OCH₃, cyclopropyl and N(CH₃)OCH₃.

According to a further preferred embodiment, also in combination with the first and third embodiment mentioned above, in the above compounds of formula (III) when R1 is methyl, R is not selected from 3-pyridyl, 4-chlorophenyl, 4-trifluoromethylphenyl, 3-tiophenyl, 3-thiazolyl-(2-methyl), phenyl, thiazole, 2-4-difluorophenyl, 4-methoxyphenyl and 2-methylthiazole.

According to another preferred embodiment, also in combination with any of the preceding embodiments, X is oxygen.

According to a further preferred embodiment, also in combination with any of the preceding embodiments, said aryl is phenyl and said heteroaryl is a 5- or 6-membered heteroaryl containing from 1 to 3 heteroatoms selected from N, O and S. Preferably, said 5- or 6- membered heteroaryl is selected from the group consisting of thiophenyl, furanyl, pyrrolyl, imidazolyl, pyrazolyl, oxadiazolyl, oxazolyl and pyridinyl.

According to a further preferred embodiment, also in combination with any of the preceding embodiments, in said compounds of formula (III), wherein R is aryl, the aryl is optionally substituted with a group selected from:
- halogen, preferably selected from Br and F;
- linear or branched C₁-C₃ alkyl, preferably CH₃;
- OR5 and NR6R7, wherein R5, R6 and R7 are independently hydrogen or linear C₁-C₃ alkyl.

Preferred identities of OR5 and NR6R7 are OH, NH₂ and NHCH₃, respectively.

According to a further preferred embodiment, also in combination with any of the preceding embodiments, in said compounds of formula (III), wherein R is heteroaryl, this is optionally substituted with linear or branched C₁-C₆ alkyl, preferably with CH₃.

Particularly preferred compounds of formula (III) for use according to the present invention are those wherein R is selected from the group consisting of 3-fluorophenyl, 4-fluorophenyl, 2-bromophenyl, 3-bromophenyl, 2-methylphenyl, 3-methylphenyl, 4-methylphenyl, 2-hydroxyphenyl, 3-hydroxyphenyl, 4-hydroxyphenyl, 3-aminophenyl, 4-aminophenyl, 3-methylaminophenyl, 4-methylaminophenyl, thiophen-2yl, furan-2yl, pyrrol-2yl, 1H-imidazol-5yl, 1-methyl-imidazol-5yl, pyrazol-4yl, 1,2,4-oxadiazol-3yl, 1,2-oxazol-5yl, pyridin-2yl, pyridin-3yl and pyridin-4yl.

Particularly preferred compounds of formula (III) for use according to the invention are selected from:
1-[2-(3-fluorophenyl)-4-hydroxy-1,3-thiazol-5-yl]propan-1-one;
sodium 2-(3-fluorophenyl)-5-propanoyl-1,3-thiazol-4-olate, also known as DFL23693;
2-(3-fluorophenyl)-4-hydroxy-N-methoxy-N-methyl-1,3-thiazole-5-carboxamide;
1-(2-(3-fluorophenyl)-4-hydroxythiazol-5-yl)ethenone;
1-[2-(3-fluorophenyl)-4-hydroxy-1,3-thiazol-5-yl]-2-methylpropan-1-one;
4-hydroxy-N-methoxy-N-methyl-2-(thiophen-2-yl)-1,3-thiazole-5-carboxamide;
1-[4-hydroxy-2-(thiophen-2-yl)-1,3-thiazol-5-yl]propan-1-one;
4-hydroxy-N-methoxy-N-methyl-2-(2-methylphenyl)-1,3-thiazole-5-carboxamide;
1-[4-hydroxy-2-(2-methylphenyl)-1,3-thiazol-5-yl]propan-1-one;
2-(2-bromophenyl)-4-hydroxy-N-methoxy-N-methyl-1,3-thiazole-5-carboxamide;
1-[2-(2-bromophenyl)-4-hydroxy-1,3-thiazol-5-yl]propan-1-one;
4-hydroxy-2-(2-hydroxyphenyl)-N-methoxy-N-methyl-1,3-thiazole-5-carboxamide;
1-[2-(2-hydroxyphenyl)-4-hydroxy-1,3-thiazol-5-yl]propan-1-one;
1-[2-(3-bromophenyl)-4-hydroxy-1,3-thiazol-5-yl]propan-1-one;
1-[2-(furan-2-yl)-4-hydroxy-1,3-thiazol-5-yl]propan-1-one;
1-[4-hydroxy-2-(1H-pyrrol-2-yl)-1 ,3-thiazol-5-yl]propan-1 -one;
1-[4-hydroxy-2-(1-methyl-1H-pyrrol-2-yl)-1,3-thiazol-5-yl]propan-1-one;
1-[4-hydroxy-2-(1-methyl-1H-imidazol-5-yl)-1,3-thiazol-5-yl]propan-1-one;
1-[4-hydroxy-2-(1H-imidazol-5-yl)-1,3-thiazol-5-yl]propan-1-one;
1-[4-hydroxy-2-(1-methyl-1H-pyrazol-4-yl)-1,3-thiazol-5-yl]propan-1-one;
1-[4-hydroxy-2-(thiophen-2-yl)-1,3-thiazol-5-yl]butan-1-one;
1-[4-hydroxy-2-(thiophen-2-yl)-1,3-thiazol-5-yl]-3-methylbutan-1-one;
1-[4-hydroxy-2-(1,2,4-oxadiazol-3-yl)-1,3-thiazol-5-yl]propan-1-one;
1-[4-hydroxy-2-(1,2-oxazol-5-yl)-1,3-thiazol-5-yl]propan-1-one;
1-[4-hydroxy-2-(pyridin-3-yl)-1,3-thiazol-5-yl]propan-1-one;
1-[4-hydroxy-2-(pyridin-4-yl)-1,3-thiazol-5-yl]propan-1-one;
1-[4-hydroxy-2-(pyridin-2-yl)-1,3-thiazol-5-yl]propan-1-one;
1-[4-hydroxy-2-(3-hydroxyphenyl)-1,3-thiazol-5-yl]propan-1-one;
1-[4-hydroxy-2-(4-hydroxyphenyl)-1,3-thiazol-5-yl]propan-1-one;
1-[4-hydroxy-2-(3-methylphenyl)-1,3-thiazol-5-yl]propan-1-one;
1-[4-hydroxy-2-(4-methylphenyl)-1,3-thiazol-5-yl]propan-1-one;
1-[2-(3-aminophenyl)-4-hydroxy-1, 3-thiazol-5-yl]propan-1-one;
1-[2-(4-aminophenyl)-4-hydroxy-1, 3-thiazol-5-yl]propan-1-one;
1-{4-hydroxy-2-[3-(methylamino)phenyl]-1,3-thiazol-5-yl}propan-1 -one;
1-{4-hydroxy-2-[4-(methylamino)phenyl]-1,3-thiazol-5-yl}propan-1-one;
1-[2-(4-fluorophenyl)-4-hydroxy-1,3-thiazol-5-yl]propan-1-one;
1-[2-(3-fluorophenyl)-4-hydroxy-1,3-thiazol-5-yl]butan-1-one;
1-[2-(3-fluorophenyl)-4-hydroxy-1,3-thiazol-5-yl]-3-methylbutan-1-one;
1-[2-(3-fluorophenyl)-4-hydroxy-1,3-thiazol-5-yl]-2-methoxyethanone;
1-[2-(3-fluorophenyl)-4-hydroxy-1,3-thiazol-5-yl]propane-1-thione;
2-(3-fluorophenyl)-4-hydroxy-*N*-methoxy-*N*-methyl-1,3-thiazole-5-carbothioamide;
2-(3-fluorophenyl)-5-[(1E)-N-methoxypropanimidoyl]-1,3-thiazol-4-ol;
2-(3-fluorophenyl)-5-propanimidoyl-1,3-thiazol-4-ol; and
2-(3-fluorophenyl)-5-[(1E)-N-hydroxypropanimidoyl]-1,3-thiazol-4-ol.

Compounds of formula (III) are disclosed in WO2015/197640A1, which also discloses their method of synthesis and their inhibitory activity towards TRPM8.

According to a further preferred embodiment, also in combination with any of the above embodiments, said TRPM8 antagonist for use according to the present invention is a compound of formula (IV) 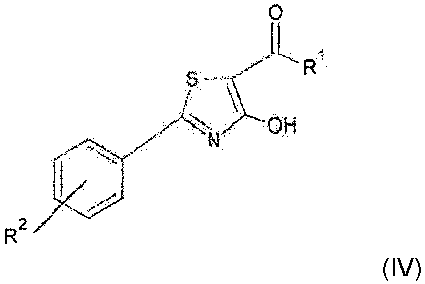
or a pharmaceutically acceptable salt thereof,
wherein
R¹ is an unsubstituted or substituted 5, 6 or 7-membered, aliphatic or aromatic heterocycle group containing 1, 2, 3 or 4 heteroatoms selected from N, O and S;
R² is selected from hydrogen, C₁-C₂ alkyl, F, Cl and OH.

The term "substituted" herein refers to mono- or poly-substitution by a named (or undefined) substituent to the extent that such a single or multiple substitution is chemically allowed.

Preferably, said 5, 6 or 7-membered, aliphatic or aromatic heterocycle is unsubstituted or substituted with one or more groups independently selected from C₁-C₃ alkyl or cycloalkyl, Cl and F. Preferably, said C₁-C₃ alkyl is methyl.

According to a preferred embodiment the heterocycle is substituted with R³ and R⁴ that taken together can form a saturated cyclic moiety, preferably cyclobutane, cyclopentane or cyclohexane.

According to a preferred embodiment the heterocycle is unsubstituted.

Preferably, R¹ is selected from the group consisting of oxazolidinyl, oxolanyl, pyrrolidinyl, oxazinanyl, morpholinyl, piperidinyl, methylpyrrolidinyl, pyrrolyl, methylpyrrolyl, furanyl, thiophenyl, pyridinyl, imidazolyl, pyrazolyl, oxadiazolyl and oxazolyl.

More preferably, R¹ is selected from the group consisting of 1,2-oxazolidin-2-yl, 1,3-oxazolidin-3-yl, oxolan-2-yl, pyrrolidin-1-yl, 1,2-oxazinan-2-yl, 1,3-oxazinan-3-yl, morpholin-4-yl, piperidin-1-yl, pyrrolidin-2-yl, 1-methylpyrrolidin-2-yl, 1H-pyrrol-2-yl, 1-methyl-1H-pyrrol-2-yl, furan-2-yl, thiophen-2-yl, 1H-pyrrol-1-yl, pyridin-2-yl, pyridin-3-yl, pyridin-4-yl, 1H-imidazol-1-yl and 1H-pyrazol-1-yl.

Preferably, R² is F or OH.

More preferably, R² is 3-F or 2-OH

In an embodiment according to the invention,
R¹ is oxazolidinyl and R² is F or OH.

In another embodiment according to the invention,
R¹ is oxolanyl and R² is F or OH.

In another embodiment according to the invention,
R¹ is pyrrolidinyl and R² is F or OH.

In another embodiment according to the invention,
R¹ is oxazinanyl and R² is F or OH.

In another embodiment according to the invention,
R¹ is morpholinyl and R² is F or OH.

In another embodiment according to the invention,
R¹ is piperidinyl and R² is F or OH.

In another embodiment according to the invention,
R¹ is methylpyrrolidinyl and R² is F or OH.

In another embodiment according to the invention,
R¹ is pyrrolyl and R² is F or OH.

In another embodiment according to the invention,
R¹ is methylpyrrolyl and R² is F or OH.

In another embodiment according to the invention,
R¹ is furanyl and R² is F or OH.

In another embodiment according to the invention,
R¹ is thiophenyl and R² is F or OH.

In another embodiment according to the invention,
R¹ is pyridinyl and R² is F or OH.

In another embodiment according to the invention,
R¹ is imidazolyl and R² is F or OH.

In another embodiment according to the invention,
R¹ is pyrazolyl and R² is F or OH.

Preferred compounds of formula (IV) for use according to the invention are selected from:
[2-(3-fluorophenyl)-4-hydroxy-1,3-thiazol-5-yl](1,2-oxazolidin-2-yl)methanone, also known as DFL23877, or the sodium salt thereof, also known as DFL24072;
[4-hydroxy-2-(2-hydroxyphenyl)-1,3-thiazol-5-yl](1,2-oxazolidin-2-yl)methanone, also known as DFL23880, or the sodium salt thereof, also known as DFL24080;
[2-(3-fluorophenyl)-4-hydroxy-1,3-thiazol-5-yl](1,3-oxazolidin-3-yl)methanone;
[4-hydroxy-2-(2-hydroxyphenyl)-1,3-thiazol-5-yl](1,3-oxazolidin-3-yl)methanone;
[2-(3-fluorophenyl)-4-hydroxy-1,3-thiazol-5-yl](oxolan-2-yl)methanone;
[4-hydroxy-2-(2-hydroxyphenyl)-1,3-thiazol-5-yl](oxolan-2-yl)methanone;
[2-(3-fluorophenyl)-4-hydroxy-1,3-thiazol-5-yl](pyrrolidin-1-yl)methanone;
[4-hydroxy-2-(2-hydroxyphenyl)-1,3-thiazol-5-yl](1,2-oxazinan-2-yl)methanone;
[2-(3-fluorophenyl)-4-hydroxy-1,3-thiazol-5-yl](1,2-oxazinan-2-yl)methanone;
[2-(3-fluorophenyl)-4-hydroxy-1,3-thiazol-5-yl](1,3-oxazinan-3-yl)methanone;
[4-hydroxy-2-(2-hydroxyphenyl)-1,3-thiazol-5-yl](1,3-oxazinan-3-yl)methanone;
[2-(3-fluorophenyl)-4-hydroxy-1,3-thiazol-5-yl](morpholin-4-yl)methanone;
[4-hydroxy-2-(2-hydroxyphenyl)-1,3-thiazol-5-yl](morpholin-4-yl)methanone;
[2-(3-fluorophenyl)-4-hydroxy-1,3-thiazol-5-yl](piperidin-1-yl)methanone;
[2-(3-fluorophenyl)-4-hydroxy-1,3-thiazol-5-yl](pyrrolidin-2-yl)methanone;
[2-(3-fluorophenyl)-4-hydroxy-1,3-thiazol-5-yl](1-methylpyrrolidin-2-yl)methanone;
[2-(3-fluorophenyl)-4-hydroxy-1,3-thiazol-5-yl](1H-pyrrol-2-yl)methanone;
[2-(3-fluorophenyl)-4-hydroxy-1,3-thiazol-5-yl](1-methyl-1H-pyrrol-2-yl)methanone;
[2-(3-fluorophenyl)-4-hydroxy-1,3-thiazol-5-yl](furan-2-yl)methanone;
[2-(3-fluorophenyl)-4-hydroxy-1,3-thiazol-5-yl](thiophen-2-yl)methanone;
[2-(3-fluorophenyl)-4-hydroxy-1,3-thiazol-5-yl](1H-pyrrol-1-yl)methanone;
[2-(3-fluorophenyl)-4-hydroxy-1,3-thiazol-5-yl](pyridin-2-yl)methanone;
[2-(3-fluorophenyl)-4-hydroxy-1,3-thiazol-5-yl](pyridin-3-yl)methanone;
[2-(3-fluorophenyl)-4-hydroxy-1,3-thiazol-5-yl](pyridin-4-yl)methanone;
[2-(3-fluorophenyl)-4-hydroxy-1,3-thiazol-5-yl](1H-imidazol-1-yl)methanone;
[2-(3-fluorophenyl)-4-hydroxy-1,3-thiazol-5-yl](1H-pyrazol-1-yl)methanone;
[2-(3-fluorophenyl)-4-hydroxy-1,3-thiazol-5-yl](1,2-oxazepan-2-yl)methanone;
[2-(3-fluorophenyl)-4-hydroxy-1,3-thiazol-5-yl](5-oxa-6-azaspiro[2.4]heptan-6-yl)methanone;
[2-(3-fluorophenyl)-4-hydroxy-1,3-thiazol-5-yl](5-methyl-1,2-oxazolidin-2-yl)methanone;
[2-(3-fluorophenyl)-4-hydroxy-1,3-thiazol-5-yl](hexahydro-2H-cyclopenta[d][1,2]oxazol-2-yl)methanone;
[2-(3-fluorophenyl)-4-hydroxy-1,3-thiazol-5-yl](1H-1,2,3-triazol-1-yl)methanone; and
[2-(3-fluorophenyl)-4-hydroxy-1,3-thiazol-5-yl](1-methyl-1H-tetrazol-5-yl)methanone.

Compounds of formula (IV) are disclosed in WO2017/108632A1, which also discloses their method of synthesis and their inhibitory activity towards TRPM8.

According to a further preferred embodiment, also in combination with any of the above embodiments, the TRPM8 inhibitor for use according to the present invention is selected from:
6-[[[(S)-(3-Fluoro-2-pyridinyl)[3-fluoro-4-(trifluoromethoxy)phenyl]methyl]amino]carbonyl]-3-pyridinecarboxylic acid, also known as AMG-333 (IC50 of 0.2 nM);
(1R)-N-(4-Fluorophenyl)-3,4-dihydro-1-[4-(trifluoromethyl)phenyl]-2(1H)-isoquinolinecarboxamide, also known as AMG-8788 (IC50 of 63.2 nM);
(1R)-3,4-Dihydro-N-[(1S)-2,2,2-trifluoro-1-methylethyl]-1-[4-(trifluoromethyl)phenyl]-2(1H)-isoquinolinecarboxamide, also known as AMG-9678 (IC50 of 31.2 nM);
N,N-Bis(phenylmethyl)-L-tryptophan methyl ester, also known as TRPM8 antagonist 2 (IC50 of 0.2 nM);
[4-Hydroxy-2-(2-hydroxyphenyl)-5-thiazolyl]-2-isoxazolidinylmethanone, also known as TRPM8 antagonist 3 (IC50 of 11 nM);
3-[[[(1R)-1-(4-Fluorophenyl)ethyl](3-quinolinylcarbonyl)amino]methyl]benzoic acid, also known as PF-05105679 (IC50 of 103 nM);
4-[[[3-Chloro-5-(trifluoromethyl)-2-pyridinyl][[4-(trifluoromethoxy)phenyl]methyl]amino]sulfonyl]benzoic acid, also known as RQ-00203078 (IC50 of 5.3 nM);
3-[7-(Trifluoromethyl)-5-[2-(trifluoromethyl)phenyl]-1H-benzim idazol-2-yl]-1-oxa-2-azaspiro[4.5]dec-2-ene, also known as TC-I-2014 (IC50 of 3 nM);
4-[[(4-Cyclopropyl-3-isoquinolinyl)[[4-(trifluoromethoxy)phenyl]methyl]amino]sulfonyl]benzoic acid, also known as MT-8554;
N-(2-aminoethyl)-N-[[3-methoxy-4-(phenylmethoxy)phenyl]methyl]-2-thiophenecarboxamide monohydrochloride, also known as M8-B;
(8R)-5,8-Dihydro-N-[(1S)-2,2,2-trifluoro-1-methylethyl]-8-[4-(trifluoromethyl)phenyl]-1,7-naphthyridine-7(6H)-carboxamide, also known as AMG-2850.

According to a particularly preferred embodiment, also in combination with any of the above embodiments, the TRPM8 inhibitor for use according to the present invention is selected from:
2-(3-fluorophenyl)-5-(2-ethyl-2H-tetrazol-5-yl)-1,3-thiazol-4-ol, also known as DFL23448;
sodium 2-(3-fluorophenyl)-5-propanoyl-1,3-thiazol-4-olate, also known as DFL23693;
2-(3-fluorophenyl)-4-hydroxy-1,3-thiazol-5-yl](1,2-oxazolidin-2-yl)methanone, also known as DFL23877, or the sodium salt thereof, also known as DFL24072; and
[4-hydroxy-2-(2-hydroxyphenyl)-1,3-thiazol-5-yl](1,2-oxazolidin-2-yl)methanone, also known as DFL23880, or the sodium salt thereof, also known as DFL24080.

According to a particularly preferred embodiment, also in combination with any of the above embodiments, the TRPM8 inhibitor for use according to the present invention is sodium 2-(3-fluorophenyl)-5-propanoyl-1,3-thiazol-4-olate, also known as DFL23693.

The TRPM8 inhibitor for use according to the present invention may form stable pharmaceutically acceptable acid or base salts with a pharmaceutically acceptable organic or inorganic acid or base, and in such cases administration of the TRPM8 inhibitor as a salt may be appropriate.

Examples of acid addition salts may include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts may include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Non-toxic physiologically acceptable salts are preferred, although other salts may be useful, such as in isolating or purifying the product. The salts may be formed by conventional means, such as by reacting the free form of the product with one or more equivalents of the appropriate acid or base in a solvent or medium in which the salt is insoluble, such as for example water or ethanol, which is removed under vacuum or by freeze drying.

Certain TRPM8 inhibitor compounds for use according to the present invention may exist in tautomeric forms, and this invention includes all such tautomeric forms of those compounds unless otherwise specified.

Unless otherwise stated, structures depicted herein are also meant to include all stereochemical forms of the structure, i.e., the R and S configurations for each asymmetric center. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as a mixture of such isomers.

Particular optical isomers can be obtained by resolution of the racemic mixtures according to conventional processes, e.g., by formation of diastereomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolysed to deliver the enantiomerically pure compound. Optically active compounds of the invention can be obtained by using active starting materials. These isomers may be in the form of a free acid, a free base, an ester or a salt.

According to a preferred embodiment, also in combination with any of the above embodiments, the TRPM8 inhibitor for use according to the present invention is administered by intra-articular injection (also known as joint injection) to the subject.

Preferably, the TRPM8 inhibitor for use according to the present invention is administered by intra-articular injection to the subject in form of a solution, which may be a liquid solution or a viscous solution.

Preferably, the TRPM8 inhibitor for use according to the present invention is administered by intra-articular injection to the subject in form of a solution which comprises a TRPM8 inhibitor, as above defined, and one or more of the following components:
polyethylene glycol (PEG), also known as polyethylene oxide (PEO), from 800,000 to 1,000,000 molecular weight (MW), preferably at an amount between 0.1 % and 2% w/V;
carboxymethyl cellulose (CMC) from 700,000 to 1,000,000 molecular weight (MW), preferably at an amount between 0.1 % and 1.0 % w/V;
polyvinylpyrrolidone (PVP) from 360 to 370 kDA, preferably at an amount between 0.1 % and 0.5 % w/V.

More preferably, the TRPM8 inhibitor for use according to the present invention is administered by intra-articular injection to the subject in form of a solution which comprises a TRPM8 inhibitor, as above defined, and one or more of the following components:
0.25 % w/V polyethylene glycol (PEG), also known as polyethylene oxide (PEO), 1,000,000 MW,
0.2 % w/V carboxymethyl cellulose (CMC) 700,000 MW,
0.2 % w/V polyvinylpyrrolidone (PVP) 365 kDA.

Preferably, the concentration of said TRPM8 inhibitor in said solution is between 50 µM and 600 µM, more preferably between 100 µM and 500 µM, even more preferably said concentration is selected from 100 µM and 500 µM. According to a preferred embodiment, also in combination with any of the above embodiments, said TRPM8 inhibitor is DFL23693.

According to a further preferred embodiment, the TRPM8 inhibitor for use according to the present invention is administered orally to the subject.

Preferably, according to this embodiment, the TRPM8 inhibitor for use according to the present invention is administered orally to the subject in form of a solution, which may be a liquid solution or a viscous solution.

Alternatively, the TRPM8 inhibitor for use according to the present invention may be administered orally to the subject in form of a solid dosage form, preferably a capsule or a tablet.

Preferably, the TRPM8 inhibitor for use according to the present invention is administered orally to the subject in form of a solution which comprises a TRPM8 inhibitor, as above defined, and the following components:
10% v/v [SOLUTOL HS-15 / N-methyl pirrolidone 2:1 (W/V)] + 90% v/v Phosphate Buffer Solution 25 mM.

SOLUTOL HS-15 is also known as Polyethylene Glycol 12-hydroxystearate. Preferably, said TRPM8 inhibitor is DFL23693.

The average daily dose of the TRPM8 inhibitor depends on several factors such as the severity of the disease, the condition, age, sex and weight of the patient. The dose will vary generally from 1 to 1000 mg of the TRPM8 inhibitor per day, optionally divided into multiple administrations.

As will be discussed in the experimental section, the present inventors have shown that oral and intra-articular administrations of a TRPM8 inhibitor using an animal model of MIA-induced osteoarthritis result in a decrease in gross pathological score of the knee joint when compared to the vehicle-treated group (oral administration) and placebo-treated group (intra-articular administration). Also, the group of animals which was treated with the highest dose tested via intra-articular injection showed a significant decrease in tibial histopathological grades, tibial inflammation scores and tibial proteoglycan scores when compared with the placebo-treated group. Collectively, these results show that the TRPM8 inhibitor preserved the cartilage and the bone of the joint from the damage and degeneration induced by the disease.

The invention is also directed to a pharmaceutical composition comprising a TRPM8 inhibitor, as defined above, and at least one pharmaceutically acceptable excipient or carrier for use in the prevention or treatment of joint damage and/or joint degeneration and/or joint inflammation caused by osteoarthritis in a subject.

According to a preferred embodiment, said pharmaceutical composition is for use in the prevention or treatment of joint damage or joint degeneration or joint inflammation caused by osteoarthritis in a subject.

According to a preferred embodiment, said pharmaceutical composition is for use in the prevention or treatment of joint damage and joint degeneration and joint inflammation caused by osteoarthritis in a subject.

According to a preferred embodiment, said pharmaceutical composition is for use in the prevention or treatment of joint damage and joint degeneration caused by osteoarthritis in a subject.

According to a preferred embodiment, said pharmaceutical composition is for use in the prevention or treatment of joint damage and joint inflammation caused by osteoarthritis in a subject.

According to a preferred embodiment, said pharmaceutical composition is for use in the prevention or treatment of joint degeneration and joint inflammation caused by osteoarthritis in a subject.

The invention is also directed to a pharmaceutical composition comprising a TRPM8 inhibitor, as defined above, and at least one pharmaceutically acceptable excipient or carrier for use in the prevention or treatment of joint damage and/or joint degeneration and/or joint inflammation in a subject with osteoarthritis.

According to a preferred embodiment, said pharmaceutical composition is for use in the prevention or treatment of joint damage or joint degeneration or joint inflammation in a subject with osteoarthritis.

According to a preferred embodiment, said pharmaceutical composition is for use in the prevention or treatment of joint damage and joint degeneration and joint inflammation in a subject with osteoarthritis.

According to a preferred embodiment, said pharmaceutical composition is for use in the prevention or treatment of joint damage and joint degeneration in a subject with osteoarthritis.

According to a preferred embodiment, said pharmaceutical composition is for use in the prevention or treatment of joint damage and joint inflammation in a subject with osteoarthritis.

According to a preferred embodiment, said pharmaceutical composition is for use in the prevention or treatment of joint degeneration and joint inflammation in a subject with osteoarthritis.

As used herein, the term "pharmaceutically acceptable excipient or carrier" includes any and all solvents, diluents, or other vehicle, dispersion or suspension aids, surface active agents, isotonic agents, surfactant, synthetic polymer, film-forming agents, plasticizers, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; cellulose and its derivatives such as sodium carboxymethyl cellulose, ethyl cellulose and cellulose acetate; malt; gelatine; oils such as peanut oil, cottonseed oil; safflower oil; sesame oil; olive oil; corn oil and soybean oil; glycols such as propylene glycol and polyethylene glycol; esters such as ethyl oleate and ethyl laurate; agar; buffering agents such as magnesium hydroxide and aluminum hydroxide; pharmaceutical carriers such us poly(vinylpyrrolidone), chitosan, polyethylene glycol; surfactants such us polyoxyethylene sorbitan monooleate, poly(vinyl alcohol), polyoxyethylene sorbitan, polyoxyethylene sorbitan monostearate, polyoxyethylene sorbitan monolaurate, poly(vinylpyrrolidone), polyethylene glycol lauryl ether, ethoxylated hydrogenated castor oil; suspending agents such us gum arabic, alginic acid, pectin, polyoxyethylene sorbitan monolaurate, guar gum, sodium alginate, α-Tocopheryl polyethylene glycol succinate, carrageenan, maltitol, gum tragacanth, pullulan; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions; organic solvents as N-methyl pyrrolidone, ethanol.

Further, the composition of the present invention may be suitably formulated using appropriate methods known in the art or by the method disclosed in Remington's Pharmaceutical Science (recent edition), Mack Publishing Company, Easton Pa.

According to a preferred embodiment, the pharmaceutical composition for use according to the present invention is suitable for oral administration. Preferably, according to this embodiment, said pharmaceutical composition for use according to the present invention is a solution, which may be a liquid solution or a viscous solution. Preferably, said solution comprises a TRPM8 inhibitor, as above defined, and the following components: 10% v/v [SOLUTOL HS-15 / N-methyl pirrolidone 2:1 (W/V)] + 90% v/v Phosphate Buffer Solution 25 mM. Preferably, said TRPM8 inhibitor is DFL23693.

Alternatively, according to this embodiment, said pharmaceutical composition for use according to the present invention is a solid dosage form, preferably a capsule or a tablet.

According to a further preferred embodiment, the pharmaceutical composition for use according to the present invention is suitable for intra-articular injection. Preferably, according to this embodiment, said pharmaceutical composition for use according to the present invention is a solution, which may be a liquid solution or a viscous solution. Preferably, said solution comprises a TRPM8 inhibitor, as above defined, and one or more of the following components:
polyethylene glycol (PEG), also known as polyethylene oxide (PEO), from 800,000 to 1,000,000 molecular weight (MW), preferably at an amount between 0.1 % and 2% w/V;
carboxymethyl cellulose (CMC) from 700,000 to 1,000,000 molecular weight (MW), preferably at an amount between 0.1 % and 1.0 % w/V;
polyvinylpyrrolidone (PVP) from 360 t0 370 kDA, preferably at an amount between 0.1 % and 0.5 % w/V.

More preferably, said solution comprises a TRPM8 inhibitor, as above defined, and one or more of the following components:
0.25 % w/V polyethylene glycol (PEG), also known as polyethylene oxide (PEO), 1,000,000 MW,
0.2 % w/V carboxymethyl cellulose (CMC) 700,000 MW,
0.2 % w/V polyvinylpyrrolidone (PVP) 365 kDA.

Preferably, the concentration of said TRPM8 inhibitor in said solution is between 50 µM and 600 µM, more preferably between 100 µM and 500 µM, even more preferably said concentration is selected from 100 µM and 500 µM. Preferably, said TRPM8 inhibitor is DFL23693.

A "therapeutically effective amount" according to the present invention means an amount sufficient to achieve treatment of the disease.

A "prophylactically effective amount" according to the present invention means an amount sufficient to achieve prevention of the disease.

Determination of the therapeutically or prophylactically effective amounts is well within the capability of those skilled in the art based upon the achievement of a desired effect. An effective amount will depend on factors including, but not limited to, the weight of a subject and/or the degree of the disease or unwanted condition from which a subject suffers.

The invention also relates to the use of a TRPM8 inhibitor, as above defined, in the manufacture of a medicament for the prevention and/or treatment of joint damage and/or joint degeneration and/or joint inflammation caused by osteoarthritis in a subject.

The invention further relates to the use of a TRPM8 inhibitor, as above defined, in the manufacture of a medicament for the prevention and/or treatment of joint damage and/or joint degeneration and/or joint inflammation in a subject with osteoarthritis.

The invention is also directed to a method of preventing and/or treating joint damage and/or joint degeneration and/or joint inflammation caused by osteoarthritis in a subject, which comprises administering an effective amount of one or more TRPM8 inhibitors, as above defined, to a subject in need thereof.

The invention is also directed to a method of preventing and/or treating joint damage and/or joint degeneration and/or joint inflammation in a subject with osteoarthritis, which comprises administering an effective amount of one or more TRPM8 inhibitors, as above defined, to a subject in need thereof.

According to a preferred embodiment, said method comprises the steps of i) selecting a subject to be treated after a clinical evaluation of the affected joint by means of an imaging technique, preferably by means of radiography, and ii) administering an effective amount of one or more TRPM8 inhibitors, as above defined, to said subject.

The invention is further illustrated by the following examples, which do not limit the scope of the invention as defined in the claims.

### EXPERIMENTAL SECTION

### EXAMPLE 1

A study was designed to evaluate the effect of administration of the TRPM8 inhibitor DFL23693 in monosodium iodoacetate (MIA) induced osteoarthritis in male Sprague Dawley (SD) rats. The rats were divided into 8 groups as summarized in the following table:

| **Group** | **Treatment** | **# Rats** | **Dose** | | **Route of Administr ation** |
|---|---|---|---|---|---|
| | | | **(mg/kg)/(µM)** | **Vol. (mL/kg)** | |
| 1 | Naive | 6 | - | 10 | *p.o*. |
| 2 | Saline, in., articl + Vehicle | 6 | - | 10 | *p*.*o*. |
| 3 | MIA+Placebo | 14 | - | 0.05 | *In., articular* |
| 4 | MIA+Vehicle | 14 | - | 10 | *p.o*. |
| 5 | MIA+Tramadol | 14 | 30 mg/kg | 6 | *p.o*. |
| 6 | MIA+ DFL23693 | 14 | 30 mg/kg | 10 | *p.o*. |
| 7 | MIA+ DFL23693 | 14 | 100 µM | 0.05 | *In., articular* |
| 8 | MIA+ DFL23693 | 14 | 500 µM | 0.05 | *In., articular* |

Two to three rats per cage were housed in standard polysulfone cages with stainless steel top grill having access for pelleted food and polycarbonate drinking water bottle with stainless steel sipper tubes. Rats were provided with clean & sterilized corn cob as bedding. Animals' post-MIA administration was maintained in soft bedding.

Certified rodent diet was provided ad libitum. Water was available ad libitum. Periodic analysis of the water was performed, and the results will be archived at the test facility. Environmental controls for the animal room were set to maintain a temperature of 22 to 25°C, humidity of 30-70% RH, and a 12-h light/12-h dark cycle.

The preparation protocol for the DFL23693 formulation, dose of 30 mpk, was carried out following the instructions in the table below:

| | |
|---|---|
| Dose level(s) | 30 mg/kg |
| Dose volume(s) per unit body | 10 mL/kg |
| Concentration (mg/mL) | 3 mg/mL |
| Vehicle | 10% v/v (Solutol HS-15/NMP 2:1, w/v) and |
| | 90% v/v phosphate buffer solution 0.1 M, pH 8.1 |
| Formulation preparation | 11.4 mg of DFL23693 was wetted with 0.380 mL of (Solutol HS-15/NMP 2:1, w/v PROVIDED BY DOMPÉ), and mixed for 15 minutes using a vortex until complete dissolution. Following, phosphate buffer solution 0.1 M (pH 8.1) was added to make up the final volume up to 3.8 mL (in portions) and mixed well. The resulting solution was clear. The final pH was 8.1. |
| | The solution is stable at room temperature up to 12h. The solution must be prepared and used on the same day. |

The following dose volumes were used for the different groups:
- The dose volume of Tramadol, 30 mpk to be administered via oral route to group 5 was 6 mL/kg.
- The dose volume of DFL23693, 100 µM or 500 µM to be administered via intra-articular injection to groups 7 & 8 was 0.05 mL/knee.
- The dose volume of DFL23693, 30 mpk to be administered via oral route to group 6 was 10 mL/kg.
- The dose volume placebo to be administered via intra-articular injection to group 3 was 0.05 mL/knee.

For induction of osteoarthritis, rats were anesthetized with isoflurane and given a single intra-articular injection of 3 mg mono-iodoacetate (MIA, Sigma) through the infrapatellar ligament of the right knee (Bove, S. E., et al., 2003, Osteoarthritis and cartilage, 11(11), 821-830; Combe R, et al., Neurosci Lett. 2004; 370(2-3):236-40). MIA was dissolved in saline and administered in a volume of 50 µL by using 1 mL BD syringe with 26-gauge, 0.5-inch needle. To ensure uniform distribution, the knee joint was flexed 5 times, and the rats were recovered.

Groups 1 to 8 received their respective treatments on days 3, 7, 14, 21 and 28 post-MIA injection.
- Group 1, naive (p.o.)
- Group 2, rats received saline, in. articular + vehicle (p.o.,)
- Group 3, animals received MIA + placebo (in-articular.,)
- Group 4, animals received MIA + vehicle (p.o.,)
- Group 5, animals received MIA + Tramadol - 30 mg/kg (p.o.,)
- Group 6, animals received MIA + DFL23693 - 30 mg/kg (p.o.,)
- Group 7, animals received MIA + DFL23693 - 100 µM (in-articular.,)
- Group 8, animals received MIA + DFL23693 - 500 µM (in-articular.,)

The body temperature was assessed delicately inserting a lubricated thermocouple probe 40 mm into each rat's rectum. The rat was positioned with all four paws on a smooth surface, gently secured by a hand over the back and the tail was lifted for the insertion of the thermocouple probe, which was left in the rectum for 40 seconds to ensure temperature stabilization. The body temperature was recorded at baseline, 0, 0.5, 3, and 7h post-test item on days 3, 7, 14, 21 and 28 post-MIA injection or on the day of dosing.

For the scoring of the knee lesion, rats were euthanized by exposure to a slowly rising concentration of CO2 on day 28 post-MIA administration. The right knee was removed and dissected, and the tibia was fixed in a 10% formalin neutral-buffered solution. The observation and scoring of knees were conducted in accordance with the method in a previous report. Briefly, the degree of lesion on the surface of the tibial head was scored on a scale of 0-4 in a blinded manner as follows: 0 = normal appearance, 1 = rough surface, 2 = moderate lesion of cartilage surface, 3 = sever lesion of subchondral bone, 4 = appearance of osteophyte formation with sever lesion of subchondral bone (Ishikawa, G., et al., 2015, Osteoarthritis and cartilage, 23(6), 925-932).

Histologic analysis (microscopic): Rats were euthanized by exposure to a solely rising concentration of CO2 on day 28 post-MIA administration. The right knee was removed and dissected, and the tibia was fixed in a 10% formalin neutral-buffered solution and subsequently decalcified with formic acid. Samples were dehydrated in an ethanol series and embedded in paraffin. Sections were stained with hematoxylin-eosin (H&E staining).

Scoring: To assess the degree of synovial inflammation a relative scoring system was used. OARSI Histopathology Grading Scale: Grade 0: Surface Intact, Cartilage morphology intact. Grade 1: Surface Intact, Grade 2: Surface Discontinuity, Grade 3: Vertical Fissures (clefts), Grade 4: Erosion, Grade 5: Denudation and Grade 6: Deformation. Inflammation Grading: Each joint was scored on a scale of 0-4 as follows: 0 = no inflammation, 1 = minimal, 2 = mild, 3 = moderate and 4 = severe/marked by a veterinary pathologist. A subsequent tissue section of 5-7 µM was stained with safranin O-fast green. Proteoglycan content was scored in safranin O-fast green stained slides on a scale of 0-4 where 0 = no loss of proteoglycan staining relative to a normal control, 1 = minimal loss, 2 = mild loss, 3 = moderate loss and 4 = total loss of proteoglycan staining. A veterinarian pathologist has been performing the histological assessment in a blind manner (Bove, S. E., et al., 2003, Osteoarthritis and cartilage, 11(11), 821-830; Pritzker, K. P., et al., 2006, Osteoarthritis and cartilage, 14(1), 13-29).

### Statistical Analysis

The data were presented as Mean ± SEM. For single variable data, one-way ANOVA was conducted followed by Sidak's multiple comparisons test. For data involving two variables, two-way ANOVA was performed followed by Dunnett's multiple comparisons test. Statistical analyses were carried out using GraphPad Prism v10.2.2.

### Effect of DFL23693 on gross pathology of knee of monosodium iodoacetate (MIA)-induced osteoarthritic pain

The results of the evaluation of gross pathology score are shown in Figure 1. DFL23693 30 mpk group exhibited a significant decrease in gross pathological score when compared with MIA + Vehicle group (Figure 1A).

DFL23693 100 µM & 500 µM treated group showed significant decrease in gross pathological score in comparison to MIA + Placebo group (Figure 1A).

This is evident from the images reported in panel B of Figure 1, which shows that the animals treated with DFL23693 had a healthier structure of the bone and of the cartilage of the knee, with less osteophyte formation, when compared with animals treated with tramadol, used as reference drug, and animals treated with placebo or vehicle.

The above results show that both oral and intra-articular administration of the TRPM8 inhibitor DFL23693 are effective in preserving the structure of the joint - specifically, both cartilage and bone - from the damage and degeneration induced by osteoarthritis.

### Effect of DFL23693 on histopathology scores of monosodium iodoacetate (MIA) -induced osteoarthritic pain.

MIA + Vehicle and MIA + Placebo exhibited a significant increase in tibial histopathological grades, tibial inflammation scores and tibial proteoglycan scores when compared with Naive group.

DFL23693 500 µM treated group showed significant decrease in tibial histopathological grades, tibial inflammation scores and tibial proteoglycan scores when compared with MIA + Placebo group.

The results are shown in Figure 2, 3 and 4.

Collectively, the above-discussed results show that the TRPM8 inhibitor preserved the cartilage and the bone of the joint from the damage, inflammation and degeneration induced by the OA disease model.

### Effect of DFL23693 on rectal temperature of monosodium iodoacetate (MIA)-induced osteoarthritic pain

No significant difference in rectal temperature was found in saline (IA) + vehicle group in comparison to naive group except day 21 at 3h (p<0.001).

Also, as can be seen in Figure 5, no significant difference in rectal temperature on experiment days was found for all the tested formulation when compared with MIA + Vehicle group (oral formulation) or MIA + Placebo group (intra-articular formulations).

Despite a clear demonstration of pharmacological activity, both in terms of an effect against joint inflammation, articular cartilage destruction and the adverse events seen, DFL23693 had no effect on core body temperature. Collectively, these data demonstrate a clear advantage of this compound during preclinical studies, strengthening its progress to the clinical phases, particularly considering the negative effect of DFL23693 on modulating the body temperature of animals.

## Claims

1. A TRPM8 inhibitor for use in the prevention or treatment of joint damage and/or joint degeneration and/or joint inflammation caused by osteoarthritis in a subject.

2. A TRPM8 inhibitor for use in the prevention or treatment of joint damage and/or joint degeneration and/or joint inflammation in a subject with osteoarthritis.

3. A TRPM8 inhibitor for use according to claim 1 or claim 2, wherein said TRMP8 inhibitor is for use in the inhibition of onset or in the delay in the onset of joint damage and/or joint degeneration and/or joint inflammation.

4. A TRPM8 inhibitor for use according to claim 1 or claim 2, wherein said TRPM8 inhibitor is for use in the inhibition or slowing of progression of joint damage and/or joint degeneration and/or joint inflammation.

5. A TRPM8 inhibitor for use according to claim 1 or claim 2, wherein said TRPM8 inhibitor is for use in the reduction of entity of joint damage and/or joint degeneration and/or joint inflammation.

6. A TRPM8 inhibitor for use according to any one of the preceding claims, wherein said TRPM8 inhibitor is for use in i) the inhibition or slowing of the formation of osteophytes, ii) the inhibition or slowing of the progression of the formation of osteophytes, or iii) the reduction of the number and/or size of osteophytes.

7. A TRPM8 inhibitor for use according to any one of the preceding claims, wherein said joint damage or degeneration is selected from joint cartilage damage or degeneration, joint bone damage or degeneration, joint cartilage and joint bone damage or degeneration, subchondral bone damage or degeneration.

8. A TRPM8 inhibitor for use according to any one of the preceding claims, wherein said osteoarthritis is selected from hand osteoarthritis, knee osteoarthritis, hip osteoarthritis, osteoarthritis of the lower back, osteoarthritis of the spine, ankle osteoarthritis, elbow osteoarthritis, osteoarthritis of the fingers, foot osteoarthritis, shoulder osteoarthritis, wrist osteoarthritis, first metatarsophalangeal (MTP) joint osteoarthritis, sacroiliac (SI) joint arthritis and cervical osteoarthritis.

9. A TRPM8 inhibitor for use according to any one of the preceding claims, wherein said TRPM8 inhibitor is an antibody selected from ACC-049, ab235890, ab314169 and NBP1-97311.

10. A TRPM8 inhibitor for use according to any one of claims 1 to 8, wherein said TRPM8 inhibitor is selected from:
a compound of formula (I): 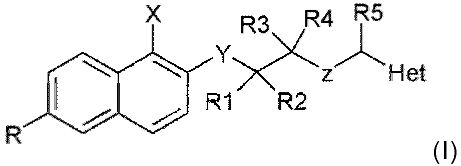
or a pharmaceutically acceptable salt thereof,
wherein
R is selected from H, Br, CN, NO₂, SO₂NH₂, SO₂NHR' and SO₂N(R')₂, where
R' is selected from linear or branched C₁-C₄ alkyl;
X is selected from F, CI, C₁-C₃ alkyl, NH₂ and OH;
Y is selected from O, CH₂, NH and SO₂;
R1 and R2, independently one from the other, are selected from H, F and linear or branched C₁-C₄ alkyl;
R₃ and R₄, independently one from the other, are selected from H and linear or branched C₁-C₄ alkyl;
Z is selected from NR6 and R6R7N⁺, where R6 and R7 independently one from the other, are selected from H and linear or branched C₁-C₄ alkyl;
R5 is a residue selected from H and linear or branched C₁-C₄ alkyl;
Het is a heteroaryl group selected from a substituted or not substituted pyrrolyl, a substituted or not substituted N- methylpyrrolyl, a substituted or not substituted thiophenyl, a substituted or not substituted furyl and a substituted or not substituted pyridinyl, and preferably it is a substituted or not substituted pyrrol-2-yl, a substituted or not substituted N- methylpyrrol-2-yl, a substituted or not substituted thiophen-2-yl, a substituted or not substituted fur-2-yl, a substituted or not substituted pyridin-2-yl;
a compound of formula (II): 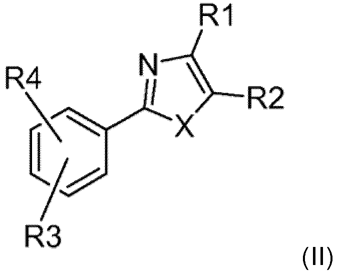
or a pharmaceutically acceptable salt thereof,
wherein
X is selected from S and O;
R1 is selected from the group consisting of:
- OR5 wherein R5 is selected from H; C₁-C₄ alkyl, trifluoromethanesulfonyl, benzyl, (trifluoromethyl)benzyl, (halo)benzyl, (trifluoromethyl)benzoyl, N-benzylcarbamoyl, cyclohexyloxyacetoyl substituted with at least one C₁-C₃ alkyl group, (C₁-C₃ alkoxy)methyl, C₁-C₃ alkanoyl and CH₂CH₂NHR6, wherein R6 is selected from H and (furan-2-yl)methyl; and
- NHR7 wherein R7 is selected from H, tert-butoxycarbonyl, C₁-C₃ alkanoyl, (4-trifluoromethyl)benzoyl, N-phenylaminoacarbonyl, CH₂R8, wherein R8 is selected from phenyl, benzo[d][1,3] dioxole, pyridin-3-yl, (pyrrolidin-1-yl) methyl, -CH2NHR9 wherein R9 is selected from H, C₁-C₃ alkyl and cycloalkyl;
R2 is selected from the group consisting of
- COOR10 wherein R10 is selected from H, C₁-C₃ alkyl and cyclohexyl, optionally substituted with at least one C₁-C₃ alkyl group;
- OH; -CONH₂; CN; -tetrazol-5-yl, 1-(C₁-C₃ alkyl)tetrazol-5-yl, 2-(C₁-C₃ alkyl)tetrazol-5-yl, 5-(C₁-C₃ alkyl)1,2,4 triazol-3-yl, 5-(C₁-C₃ alkyl)1,2,4-oxadiazol-3yl, 5-(C₁-C₃ alkyl) 1,3,4-oxadiazol-2-yl;
R3 is selected from F and H,
R4 is selected from H; CH₃; halogen; dimethylamino; pyridin-4yl; phenyl; 2- or 4- (halo)phenyl; 2- or 4- (trifluoromethyl)phenyl; 2- and/or 4-halobenzyloxy;
a compound of formula (III): 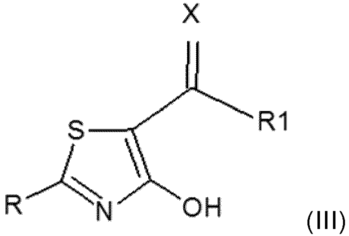
or a pharmaceutically acceptable salt thereof,
wherein
X is oxygen, sulphur, NH, NOH, or NOMe;
R is a group selected from aryl and heteroaryl, optionally substituted by one or more substituents selected from
- hydrogen,
- halogen,
- CF₃,
- linear or branched C₁-C₆ alkyl,
- OR5 and
- NR6R7, wherein R5, R6 and R7 are independently hydrogen or linear or branched C₁-C₆ alkyl;
R1 is a group selected from
- linear or branched C₁-C₆ alkyl,
- (CH₂)ₘ-OR2, wherein m is an integer between 1 and 3 and R2 is selected from hydrogen and linear C₁-C₃ alkyl,
- C₃-C₆ cycloalkyl, and
- N(R3)OR4, wherein R3 and R4 are independently hydrogen or linear or branched C₁-C₃ alkyl;
a compound of formula (IV): 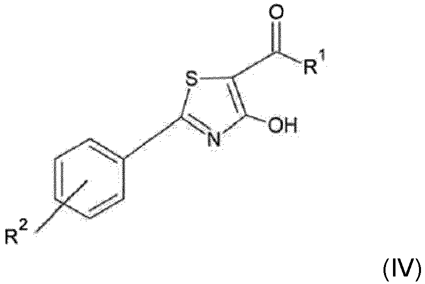
or a pharmaceutically acceptable salt thereof,
wherein
R¹ is an unsubstituted or substituted 5, 6 or 7-membered, aliphatic or aromatic heterocycle group containing 1, 2, 3 or 4 heteroatoms selected from N, O and S;
R² is selected from hydrogen, C₁-C₂ alkyl, F, Cl and OH;
6-[[[(S)-(3-Fluoro-2-pyridinyl)[3-fluoro-4-(trifluoromethoxy)phenyl]methyl]amino]carbonyl]-3-pyridinecarboxylic acid, also known as AMG-333;
(1R)-N-(4-Fluorophenyl)-3,4-dihydro-1-[4-(trifluoromethyl)phenyl]-2(1H)-isoquinolinecarboxamide, also known as AMG-8788;
(1R)-3,4-Dihydro-N-[(1S)-2,2,2-trifluoro-1-methylethyl]-1-[4-(trifluoromethyl)phenyl]-2(1H)-isoquinolinecarboxamide, also known as AMG-9678;
N,N-Bis(phenylmethyl)-L-tryptophan methyl ester, also known as TRPM8 antagonist 2;
[4-Hydroxy-2-(2-hydroxyphenyl)-5-thiazolyl]-2-isoxazolidinylmethanone, also known as TRPM8 antagonist 3;
3-[[[(1 R)-1-(4-Fluorophenyl)ethyl](3-quinolinylcarbonyl)amino]methyl]benzoic acid, also known as PF-05105679;
4-[[[3-Chloro-5-(trifluoromethyl)-2-pyridinyl][[4-(trifluoromethoxy)phenyl]methyl]amino]sulfonyl]benzoic acid, also known as RQ-00203078;
3-[7 -(Trifluoromethyl)-5-[2-(trifluoromethyl)phenyl]-1H-benzim idazol-2-yl]-1-oxa-2-azaspiro[4.5]dec-2-ene, also known as TC-I-2014;
4-[[(4-Cyclopropyl-3-isoquinolinyl)[[4-(trifluoromethoxy)phenyl]methyl]amino]sulfonyl]benzoic acid, also known as MT-8554;
N-(2-aminoethyl)-N-[[3-methoxy-4-(phenylmethoxy)phenyl]methyl]-2-thiophenecarboxamide monohydrochloride, also known as M8-B;
(8R)-5,8-Dihydro-N-[(1S)-2,2,2-trifluoro-1-methylethyl]-8-[4-(trifluoromethyl)phenyl]-1,7-naphthyridine-7(6H)-carboxamide, also known as AMG-2850.

11. A TRPM8 inhibitor for use according to claim 10, wherein said compound of formula (II) is selected from:
2-(4-chlorophenyl)-4-hydroxy-1,3-thiazole-5-carboxylic acid;
4-hydroxy-2-(4-methylphenyl)-1,3-thiazole-5-carboxylic acid;
2-(3-fluorophenyl)-4-hydroxy-1,3-thiazole-5-carboxylic acid;
2-(4-fluorophenyl)-4-hydroxy-1,3-thiazole-5-carboxylic acid;
methyl 4-hydroxy-2-phenyl-1,3-thiazole-5-carboxylate;
methyl 2-(2,4-difluorophenyl)-4-hydroxy-1,3-thiazole-5-carboxylate;
ethyl 4-hydroxy-2-phenyl-1,3-thiazole-5-carboxylate;
ethyl 2-(4-chlorophenyl)-4-hydroxy-1,3-thiazole-5-carboxylate;
ethyl 4-hydroxy-2-(4-methylphenyl)-1,3-thiazole-5-carboxylate;
ethyl 2-(3-fluorophenyl)-4-hydroxy-1,3-thiazole-5-carboxylate;
ethyl 2-(4-fluorophenyl)-4-hydroxy-1,3-thiazole-5-carboxylate;
ethyl 4-hydroxy-2-(pyridin-4-yl)-1,3-thiazole-5-carboxylate;
ethyl 2-[4-(dimethylamino)phenyl]-4-hydroxy-1,3-thiazole-5-carboxylate;
ethyl 2-(3-chlorophenyl)-4-hydroxy-1,3-thiazole-5-carboxylate;
ethyl 4-hydroxy-2-[2'-(trifluoromethyl)biphenyl-3-yl]-1,3-thiazole-5-carboxylate;
ethyl 2-(2'-fluorobiphenyl-3-yl)-4-hydroxy-1,3-thiazole-5-carboxylate;
ethyl 4-hydroxy-2-[2'-(trifluoromethyl)biphenyl-4-yl]-1,3-thiazole-5-carboxylate;
ethyl 2-(2'-fluorobiphenyl-4-yl)-4-hydroxy-1,3-thiazole-5-carboxylate;
ethyl 2-{4-[(2-fluorobenzyl)oxy]phenyl}-4-hydroxy-1,3-thiazole-5-carboxylate;
ethyl 2-{4-[(4-fluorobenzyl)oxy]phenyl}-4-hydroxy-1,3-thiazole-5-carboxylate;
ethyl 2-(4-fluorophenyl)-4-{[(trifluoromethyl)sulfonyl]oxy}-1,3-thiazole-5-carboxylate;
ethyl 4-methoxy-2-(4-methylphenyl)-1,3-thiazole-5-carboxylate;
ethyl 2-(4-methylphenyl)-4-(2-methylpropoxy)-1,3-thiazole-5-carboxylate;
ethyl 4-(benzyloxy)-2-phenyl-1,3-thiazole-5-carboxylate;
ethyl 4-[(3-chlorobenzyl)oxy]-2-(4-chlorophenyl)-1,3-thiazole-5-carboxylate;
ethyl 4-[(3-chlorobenzyl)oxy]-2-(3-fluorophenyl)-1,3-thiazole-5-carboxylate;
ethyl 4-[(4-chlorobenzyl)oxy]-2-phenyl-1,3-thiazole-5-carboxylate;
ethyl 4-[(4-chlorobenzyl)oxy]-2-(3-chlorophenyl)-1,3-thiazole-5-carboxylate;
ethyl 4-[(4-chlorobenzyl)oxy]-2-(4-methylphenyl)-1,3-thiazole-5-carboxylate;
ethyl 4-[(4-chlorobenzyl)oxy]-2-(3-fluorophenyl)-1,3-thiazole-5-carboxylate;
ethyl 4-[(2-chlorobenzyl)oxy]-2-phenyl-1,3-thiazole-5-carboxylate;
ethyl 4-[(2-chlorobenzyl)oxy]-2-(4-fluorophenyl)-1,3-thiazole-5-carboxylate;
ethyl 4-[(2-chlorobenzyl)oxy]-2-(4-chlorophenyl)-1,3-thiazole-5-carboxylate;
ethyl 4-[(2-chlorobenzyl)oxy]-2-(3-chlorophenyl)-1,3-thiazole-5-carboxylate;
ethyl 4-[(2-chlorobenzyl)oxy]-2-(4-methylphenyl)-1,3-thiazole-5-carboxylate;
ethyl 2-phenyl-4-{[4-(trifluoromethyl)benzoyl]oxy}-1,3-thiazole-5-carboxylate;
ethyl 2-(3-fluorophenyl)-4-{[4-(trifluoromethyl)benzoyl]oxy}-1,3-thiazole-5-carboxylate;
ethyl 2-(4-methylphenyl)-4-{[4-(trifluoromethyl)benzoyl]oxy}-1,3-thiazole-5-carboxylate;
ethyl 4-(2-((1R,2S,5R)-2-isopropyl-5-methylcyclohexyloxy)acetoyloxy)-2-(4-methylphenyl)-1,3-thiazole-5-carboxylate;
ethyl 4-[(benzylcarbamoyl)oxy]-2-(4-chlorophenyl)-1,3-thiazole-5-carboxylate;
ethyl 4-(2-aminoethoxy)-2-(4-chlorophenyl)-1,3-thiazole-5-carboxylate;
ethyl 2-(4-chlorophenyl)-4-{2-[(furan-2-ylmethyl)amino]ethoxy}-1,3-thiazole-5-carboxylate;
4-[(4-chlorobenzyl)oxy]-2-(4-methylphenyl)-1,3-thiazole-5-carboxylic acid;
4-[(4-chlorobenzyl)oxy]-2-phenyl-1,3-thiazole-5-carboxylic acid;
4-[(4-chlorobenzyl)oxy]-2-(4-chlorophenyl)-1,3-thiazole-5-carboxylic acid;
4-[(4-chlorobenzyl)oxy]-2-(3-chlorophenyl)-1,3-thiazole-5-carboxylic acid;
4-(benzyloxy)-2-phenyl-1,3-thiazole-5-carboxylic acid;
4-[(3-chlorobenzyl)oxy]-2-(3-fluorophenyl)-1,3-thiazole-5-carboxylic acid;
4-[(2-chlorobenzyl)oxy]-2-phenyl-1,3-thiazole-5-carboxylic acid;
4-[(2-chlorobenzyl)oxy]-2-(4-fluorophenyl)-1,3-thiazole-5-carboxylic acid;
4-[(2-chlorobenzyl)oxy]-2-(4-chlorophenyl)-1,3-thiazole-5-carboxylic acid;
4-[(2-chlorobenzyl)oxy]-2-(3-chlorophenyl)-1,3-thiazole-5-carboxylic acid;
4-[(2-chlorobenzyl)oxy]-2-(4-methylphenyl)-1,3-thiazole-5-carboxylic acid;
4-[(2-chlorobenzyl)oxy]-2-(3-fluorophenyl)-1,3-thiazole-5-carboxylic acid;
2-phenyl-4-{[4-(trifluoromethyl)benzyl]oxy}-1,3-thiazole-5-carboxylic acid;
2-(3-fluorophenyl)-4-{[4-(trifluoromethyl)benzyl]oxy}-1,3-thiazole-5-carboxylic acid;
2-phenyl-4-{[4-(trifluoromethyl)benzoyl]oxy}-1,3-thiazole-5-carboxylic acid;
2-(3-fluorophenyl)-4-{[4-(trifluoromethyl)benzoyl]oxyl-1 3-thiazole-5-carboxylic acid;
2-(4-methylphenyl)-4-{[4-(trifluoromethyl)benzoyl]oxy}-1,3-thiazole-5-carboxylic acid;
4-methoxy-2-(4-methylphenyl)-1,3-thiazole-5-carboxylic acid;
2-(4-methylphenyl)-4-(2-methylpropoxy)-1,3-thiazole-5-carboxylic acid;
ethyl 4-[(tert-butoxycarbonyl)amino]-2-(4-fluorophenyl)-1,3-thiazole-5-carboxylate;
ethyl 4-amino-2-(4-fluorophenyl)-1,3-thiazole-5-carboxylate hydrochloride;
ethyl 4-(acetylamino)-2-(4-methylphenyl)-1,3-thiazole-5-carboxylate;
ethyl 2-(4-methylphenyl)-4-{[4-(trifluoromethyl)benzoyl]amino}-1,3-thiazole-5-carboxylate;
ethyl 2-(4-methylphenyl)-4-[(phenylcarbamoyl)amino]-1,3-thiazole-5-carboxylate;
ethyl 4-[(2-aminoethyl)amino]-2-(4-methylphenyl)-1, 3-thiazole-5-carboxylate;
ethyl 2-(4-chlorophenyl)-4-{[2-(methylamino)ethyl]amino}-1,3-thiazole-5-carboxylate;
ethyl 2-(4-chlorophenyl)-4-{[2-(propylamino)ethyl]amino}-1,3-thiazole-5-carboxylate;
ethyl 4-[(2-aminoethyl)amino]-2-(4-chlorophenyl)-1,3-thiazole-5-carboxylate;
ethyl 4-{[2-(methylamino)ethyl]amino}-2-(4-methylphenyl)-1,3-thiazole-5-carboxylate;
ethyl 4-[(2-aminoethyl)amino]-2-[2'-(trifluoromethyl)biphenyl-4-yl]-1,3-thiazole-5-carboxylate;
ethyl 4-[(2-aminoethyl)amino]-2-[2'-(trifluoromethyl)biphenyl-3-yl]-1,3-thiazole-5-carboxylate;
ethyl 2-(4-chlorophenyl)-4-{[2-(cyclopentylamino)ethyl]amino}-1,3-thiazole-5-carboxylate;
ethyl 2-phenyl-4-{[2-(pyrrolidin-1-yl)ethyl]amino}-1,3-thiazole-5-carboxylate;
ethyl 4-(benzylamino)-2-(3-fluorophenyl)-1,3-thiazole-5-carboxylate;
ethyl 4-[(1,3-benzodioxol-5-ylmethyl)amino]-2-(3-fluorophenyl)-1,3-thiazole-5-carboxylate;
ethyl 2-(3-fluorophenyl)-4-[(pyridin-3-ylmethyl)amino]-1,3-thiazole-5-carboxylate;
4-[(2-aminoethyl)amino]-2-(4-methylphenyl)-1,3-thiazole-5-carboxylic acid;
4-{[2-(methylamino)ethyl]amino}-2-(4-methylphenyl)-1,3-thiazole-5-carboxylic acid;
4-[(2-aminoethyl)amino]-2-(3-fluorophenyl)-1,3-thiazole-5-carboxylic acid;
sodium 4-[(3-chlorobenzyl)oxy]-2-(3-fluorophenyl)-1,3-thiazole-5-carboxylate;
sodium 4-[(4-chlorobenzyl)oxy]-2-(4-methylphenyl)-1,3-thiazole-5-carboxylate;
sodium 4-(4-chlorobenzyloxy)-2-(4-chlorophenyl)-1,3-thiazole-5-carboxylate;
sodium 4-(2-chlorobenzyloxy)-2-(4-chlorophenyl)-1,3-thiazole-5-carboxylate;
sodium 4-(2-chlorobenzyloxy)-2-(4-methylphenyl)-1,3-thiazole-5-carboxylate;
sodium 4-(2-chlorobenzyloxy)-2-(3-fluorophenyl)-1,3-thiazole-5-carboxylate;
sodium 4-(4-chlorobenzyloxy)-2-(3-fluorophenyl)-1,3-thiazole-5-carboxylate;
(1R,2S,5R)-2-isopropyl-5-methylcyclohexyl-4-(benzyloxy)-2-(4-methylphenyl)-1,3-thiazole-5-carboxylate;
(1R,2S,5R)-2-isopropyl-5-methylcyclohexyl-4-hydroxy-2-(4-methylphenyl)-1,3-thiazole-5-carboxylate;
ethyl 2-(4-chlorophenyl)-4-(methoxymethoxy)-1,3-thiazole-5-carboxylate;
2-(4-chlorophenyl)-4-(methoxymethoxy)-1,3-thiazole-5-carboxylic acid;
2-(4-chlorophenyl)-4-(methoxymethoxy)-1,3-thiazole-5-carboxamide;
2-(4-chlorophenyl)-4-(methoxymethoxy)-1,3-thiazole-5-carbonitrile;
2-(4-chlorophenyl)-5-(1H-tetrazol-5-yl)-1,3-thiazol-4-ol;
2-(4-chlorophenyl)-5-(1-methyl-1H-tetrazol-5-yl)-1,3-thiazol-4-ol;
2-(3-fluorophenyl)-5-(1-methyl-1H-tetrazol-5-yl)-1,3-thiazol-4-ol;
2-(4-chlorophenyl)-5-(5-methyl-4H-1,2,4-triazol-3-yl)-1,3-thiazol-4-ol;
2-(3-fluorophenyl)-5-(5-methyl-4H-1,2,4-triazol-3-yl)-1,3-thiazol-4-ol;
2-(4-chlorophenyl)-5-(5-methyl-1,2,4-oxadiazol-3-yl)-1,3-thiazol-4-ol;
2-(3-fluorophenyl)-5-(5-methyl-1,2,4-oxadiazol-3-yl)-1,3-thiazol-4-ol;
3-{4-[(4-chlorobenzyl)oxy]-2-(3-fluorophenyl)-1,3-thiazol-5-yl}-5-methyl-1,2,4-oxadiazole;
2-(4-chlorophenyl)-5-(5-methyl-1,3,4-oxadiazol-2-yl)-1,3-thiazol-4-ol;
2-(3-fluorophenyl)-5-(5-methyl-1,3,4-oxadiazol-2-yl)-1,3-thiazol-4-ol;
ethyl 4-hydroxy-2-phenyl-1,3-oxazole-5-carboxylate;
ethyl 2-(3-fluorophenyl)-4-hydroxy-1,3-oxazole-5-carboxylate;
ethyl 4-hydroxy-2-(4-methylphenyl)-1,3-oxazole-5-carboxylate;
ethyl 4-[(4-chlorobenzyl)oxy]-2-phenyl-1,3-oxazole-5-carboxylate;
ethyl 4-[(4-chlorobenzyl)oxy]-2-(3-fluorophenyl)-1,3-oxazole-5-carboxylate;
ethyl 4-[(4-chlorobenzyl)oxy]-2-(4-methylphenyl)-1,3-oxazole-5-carboxylate;
ethyl 2-phenyl-4-{[4-(trifluoromethyl)benzoyl]oxy}-1 ,3-oxazole-5-carboxylate;
4-[(4-chlorobenzyl)oxy]-2-phenyl-1,3-oxazole-5-carboxylic acid;
4-[(4-chlorobenzyl)oxy]-2-(3-fluorophenyl)-1,3-oxazole-5-carboxylic acid;
4-[(4-chlorobenzyl)oxy]-2-(4-methylphenyl)-1,3-oxazole-5-carboxylic acid;
2-(3-fluorophenyl)-5-(5-methyl-1,2,4-oxadiazol-3-yl)-1,3-oxazol-4-ol;
3-{4-[(4-chlorobenzyl)oxy]-2-(3-fluorophenyl)-1,3-oxazol-5-yl}-5-methyl-1,2,4-oxadiazole;
ethyl 2-(3-fluorophenyl)-5-hydroxy-1 ,3-thiazole-4-carboxylate; and
2-(3-fluorophenyl)-5-(2-ethyl-2H-tetrazol-5-yl)-1,3-thiazol-4-ol, also known as DFL23448.

12. A TRPM8 inhibitor for use according to claim 10, wherein said compound of formula (III) is selected from:
1-[2-(3-fluorophenyl)-4-hydroxy-1,3-thiazol-5-yl]propan-1-one;
sodium 2-(3-fluorophenyl)-5-propanoyl-1,3-thiazol-4-olate, also known as DFL23693;
2-(3-fluorophenyl)-4-hydroxy-N-methoxy-N-methyl-1,3-thiazole-5-carboxamide;
1-(2-(3-fluorophenyl)-4-hydroxythiazol-5-yl)ethenone;
1-[2-(3-fluorophenyl)-4-hydroxy-1,3-thiazol-5-yl]-2-methylpropan-1-one;
4-hydroxy-N-methoxy-N-methyl-2-(thiophen-2-yl)-1, 3-thiazole-5-carboxam ide;
1-[4-hydroxy-2-(thiophen-2-yl)-1,3-thiazol-5-yl]propan-1-one;
4-hydroxy-N-methoxy-N-methyl-2-(2-methylphenyl)-1,3-thiazole-5-carboxamide;
1-[4-hydroxy-2-(2-methylphenyl)-1,3-thiazol-5-yl]propan-1-one;
2-(2-bromophenyl)-4-hydroxy-N-methoxy-N-methyl-1,3-thiazole-5-carboxamide;
1-[2-(2-bromophenyl)-4-hydroxy-1,3-thiazol-5-yl]propan-1-one;
4-hydroxy-2-(2-hydroxyphenyl)-N-methoxy-N-methyl-1,3-thiazole-5-carboxamide;
1-[2-(2-hydroxyphenyl)-4-hydroxy-1,3-thiazol-5-yl]propan-1-one;
1-[2-(3-bromophenyl)-4-hydroxy-1,3-thiazol-5-yl]propan-1-one;
1-[2-(furan-2-yl)-4-hydroxy-1,3-thiazol-5-yl]propan-1-one;
1-[4-hydroxy-2-(1H-pyrrol-2-yl)-1,3-thiazol-5-yl]propan-1 -one;
1-[4-hydroxy-2-(1-methyl-1H-pyrrol-2-yl)-1,3-thiazol-5-yl]propan-1-one;
1-[4-hydroxy-2-(1-methyl-1H-imidazol-5-yl)-1,3-thiazol-5-yl]propan-1-one;
1-[4-hydroxy-2-(1H-imidazol-5-yl)-1,3-thiazol-5-yl]propan-1-one;
1-[4-hydroxy-2-(1-methyl-1H-pyrazol-4-yl)-1,3-thiazol-5-yl]propan-1-one;
1-[4-hydroxy-2-(thiophen-2-yl)-1,3-thiazol-5-yl]butan-1-one;
1-[4-hydroxy-2-(thiophen-2-yl)-1,3-thiazol-5-yl]-3-methylbutan-1-one;
1-[4-hydroxy-2-(1,2,4-oxadiazol-3-yl)-1,3-thiazol-5-yl]propan-1-one;
1-[4-hydroxy-2-(1,2-oxazol-5-yl)-1,3-thiazol-5-yl]propan-1-one;
1-[4-hydroxy-2-(pyridin-3-yl)-1,3-thiazol-5-yl]propan-1-one;
1-[4-hydroxy-2-(pyridin-4-yl)-1,3-thiazol-5-yl]propan-1-one;
1-[4-hydroxy-2-(pyridin-2-yl)-1,3-thiazol-5-yl]propan-1-one;
1-[4-hydroxy-2-(3-hydroxyphenyl)-1,3-thiazol-5-yl]propan-1-one;
1-[4-hydroxy-2-(4-hydroxyphenyl)-1,3-thiazol-5-yl]propan-1-one;
1-[4-hydroxy-2-(3-methylphenyl)-1,3-thiazol-5-yl]propan-1-one;
1-[4-hydroxy-2-(4-methylphenyl)-1,3-thiazol-5-yl]propan-1-one;
1-[2-(3-aminophenyl)-4-hydroxy-1,3-thiazol-5-yl]propan-1-one;
1-[2-(4-aminophenyl)-4-hydroxy-1,3-thiazol-5-yl]propan-1-one;
1-{4-hydroxy-2-[3-(methylamino)phenyl]-1,3-thiazol-5-yl}propan-1-one;
1-{4-hydroxy-2-[4-(methylamino)phenyl]-1,3-thiazol-5-yl}propan-1-one;
1-[2-(4-fluorophenyl)-4-hydroxy-1,3-thiazol-5-yl]propan-1-one;
1-[2-(3-fluorophenyl)-4-hydroxy-1,3-thiazol-5-yl]butan-1-one;
1-[2-(3-fluorophenyl)-4-hydroxy-1,3-thiazol-5-yl]-3-methylbutan-1-one;
1-[2-(3-fluorophenyl)-4-hydroxy-1,3-thiazol-5-yl]-2-methoxyethanone;
1-[2-(3-fluorophenyl)-4-hydroxy-1,3-thiazol-5-yl]propane-1-thione;
2-(3-fluorophenyl)-4-hydroxy-N-methoxy-N-methyl-1, 3-thiazole-5-carbothioam ide;
2-(3-fluorophenyl)-5-[(1E)-N-methoxypropanimidoyl]-1,3-thiazol-4-ol;
2-(3-fluorophenyl)-5-propanimidoyl-1,3-thiazol-4-ol; and
2-(3-fluorophenyl)-5-[(1E)-N-hydroxypropanimidoyl]-1,3-thiazol-4-ol.

13. A TRPM8 inhibitor for use according to claim 10, wherein said compound of formula (IV) is selected from:
[2-(3-fluorophenyl)-4-hydroxy-1,3-thiazol-5-yl](1,2-oxazolidin-2-yl)methanone, also known as DFL23877, or the sodium salt thereof, also known as DFL24072;
[4-hydroxy-2-(2-hydroxyphenyl)-1,3-thiazol-5-yl](1,2-oxazolidin-2-yl)methanone, also known as DFL23880, or the sodium salt thereof, also known as DFL24080;
[2-(3-fluorophenyl)-4-hydroxy-1,3-thiazol-5-yl](1,3-oxazolidin-3-yl)methanone;
[4-hydroxy-2-(2-hydroxyphenyl)-1,3-thiazol-5-yl](1,3-oxazolidin-3-yl)methanone;
[2-(3-fluorophenyl)-4-hydroxy-1,3-thiazol-5-yl](oxolan-2-yl)methanone;
[4-hydroxy-2-(2-hydroxyphenyl)-1,3-thiazol-5-yl](oxolan-2-yl)methanone;
[2-(3-fluorophenyl)-4-hydroxy-1,3-thiazol-5-yl](pyrrolidin-1-yl)methanone;
[4-hydroxy-2-(2-hydroxyphenyl)-1,3-thiazol-5-yl](1,2-oxazinan-2-yl)methanone;
[2-(3-fluorophenyl)-4-hydroxy-1,3-thiazol-5-yl](1,2-oxazinan-2-yl)methanone;
[2-(3-fluorophenyl)-4-hydroxy-1,3-thiazol-5-yl](1,3-oxazinan-3-yl)methanone;
[4-hydroxy-2-(2-hydroxyphenyl)-1,3-thiazol-5-yl](1,3-oxazinan-3-yl)methanone;
[2-(3-fluorophenyl)-4-hydroxy-1,3-thiazol-5-yl](morpholin-4-yl)methanone;
[4-hydroxy-2-(2-hydroxyphenyl)-1,3-thiazol-5-yl](morpholin-4-yl)methanone;
[2-(3-fluorophenyl)-4-hydroxy-1,3-thiazol-5-yl](piperidin-1-yl)methanone;
[2-(3-fluorophenyl)-4-hydroxy-1,3-thiazol-5-yl](pyrrolidin-2-yl)methanone;
[2-(3-fluorophenyl)-4-hydroxy-1,3-thiazol-5-yl](1-methylpyrrolidin-2-yl)methanone;
[2-(3-fluorophenyl)-4-hydroxy-1,3-thiazol-5-yl](1H-pyrrol-2-yl)methanone;
[2-(3-fluorophenyl)-4-hydroxy-1 ,3-thiazol-5-yl](1 -methyl-1 H-pyrrol-2-yl)methanone;
[2-(3-fluorophenyl)-4-hydroxy-1,3-thiazol-5-yl](furan-2-yl)methanone;
[2-(3-fluorophenyl)-4-hydroxy-1,3-thiazol-5-yl](thiophen-2-yl)methanone;
[2-(3-fluorophenyl)-4-hydroxy-1,3-thiazol-5-yl](1H-pyrrol-1-yl)methanone;
[2-(3-fluorophenyl)-4-hydroxy-1,3-thiazol-5-yl](pyridin-2-yl)methanone;
[2-(3-fluorophenyl)-4-hydroxy-1,3-thiazol-5-yl](pyridin-3-yl)methanone;
[2-(3-fluorophenyl)-4-hydroxy-1,3-thiazol-5-yl](pyridin-4-yl)methanone;
[2-(3-fluorophenyl)-4-hydroxy-1,3-thiazol-5-yl](1H-imidazol-1-yl)methanone;
[2-(3-fluorophenyl)-4-hydroxy-1,3-thiazol-5-yl](1H-pyrazol-1-yl)methanone;
[2-(3-fluorophenyl)-4-hydroxy-1,3-thiazol-5-yl](1,2-oxazepan-2-yl)methanone;
[2-(3-fluorophenyl)-4-hydroxy-1,3-thiazol-5-yl](5-oxa-6-azaspiro[2.4]heptan-6-yl)methanone;
[2-(3-fluorophenyl)-4-hydroxy-1,3-thiazol-5-yl](5-methyl-1,2-oxazolidin-2-yl)methanone;
[2-(3-fluorophenyl)-4-hydroxy-1,3-thiazol-5-yl](hexahydro-2H-cyclopenta[d][1,2]oxazol-2-yl)methanone;
[2-(3-fluorophenyl)-4-hydroxy-1,3-thiazol-5-yl](1H-1,2,3-triazol-1-yl)methanone; and
[2-(3-fluorophenyl)-4-hydroxy-1,3-thiazol-5-yl](1-methyl-1H-tetrazol-5-yl)methanone.

14. The TRPM8 inhibitor for use according to any one of the preceding claims, wherein said TRPM8 inhibitor is administered by intra-articular injection to the subject.

15. The TRPM8 inhibitor for use according to any one of claims 1-13, wherein said TRPM8 inhibitor is administered orally to the subject.
